# EUROPÄISCHE PATENTSCHRIFT

(11) **EP 3 403 072 B1**
(45) Veröffentlichungstag und Bekanntmachung des Hinweises auf die Patenterteilung: **14.08.2024**
(21) Anmeldenummer: 17701431.3
(22) Anmeldetag: 12.01.2017
(51) Int. Cl.: G01N 21/64, G01N 33/00, G01N 21/77

(54) **PORTABLES GERÄT ZUR DETEKTION VON EXPLOSIVSTOFFEN MIT EINER VORRICHTUNG ZUR ERZEUGUNG UND MESSUNG VON EMISSION EINES INDIKATORS**
PORTABLE DEVICE FOR DETECTING EXPLOSIVE SUBSTANCES COMPRISING A DEVICE FOR GENERATING AND MEASURING THE EMISSION OF AN INDICATOR
APPAREIL PORTATIF POUR DÉTECTER DES EXPLOSIFS AVEC UN DISPOSITIF POUR PRODUIRE ET MESURER DES ÉMISSIONS D'UN INDICATEUR

(30) Priorität: 13.01.2016 DE 102016200271
(43) Veröffentlichungstag der Anmeldung: 21.11.2018
(73) Patentinhaber: Institut Dr. Foerster GmbH & Co. KG, 72766 Reutlingen (DE)
(72) Erfinder: WIDMANN, Stefan, 72379 Hechingen (DE); SINN, Gert, 12045 Berlin (DE); MITTENZWEY, Klaus-Henrik, 10439 Berlin (DE)
(74) Vertreter: Zimmermann & Partner Patentanwälte mbB
(86) Internationale Anmeldenummer: PCT/EP2017/050590
(87) Internationale Veröffentlichungsnummer: WO 2017/121814

(56) Entgegenhaltungen:
- WO-A1-2010/082852
- WO-A1-2015/191510
- KR-A- 20030 040 830
- US-A1- 2004 063 168
- US-A1- 2004 106 211
- US-A1- 2006 109 475
- US-A1- 2007 243 107
- XIN YUNHONG ET AL: "A portable fluorescence detector for fast ultra trace detection of explosive vapors", REVIEW OF SCIENTIFIC INSTRUMENTS, AIP, MELVILLE, NY, US, vol. 82, no. 10, 1 October 2011 (2011-10-01), pages 103102 - 103102, XP012152051, ISSN: 0034-6748, [retrieved on 20111004], DOI: 10.1063/1.3642661

## Beschreibung

### ANWENDUNGSGEBIET UND STAND DER TECHNIK

Die Erfindung betrifft ein portables Gerät zur Detektion von Explosivstoffen mit einer Vorrichtung zur Erzeugung und Messung von Emission eines Indikators.

Die Emission kann beispielsweise durch Anregung mit Strahlung (Fluoreszenz, Phosphoreszenz) oder durch eine chemische Reaktion (Chemilumineszenz) erzeugt werden. Im Fokus stehen die Erzeugung und Messung der Emission von Indikatorsubstanzen, die auf einem Träger aufgebracht sind. Ein Träger kann z.B. ein Glas oder ein Kunststoff sein, auf dem die Indikatorsubstanzen (eine oder mehrere) als dünne Schicht aufgebracht sind. Indikatorsubstanzen können z.B. fluoreszenzfähige Polymermoleküle sein, deren Fluoreszenz sich nach einer Kontaktierung mit bestimmten Analyten ändert. Analyten sind Verbindungen, Stoffe oder Substanzen, über die bei einer Messung eine Aussage gemacht werden soll. Die Änderung der Fluoreszenz der Indikatorsubstanz ist ein Hinweis auf die Existenz des Analyten oder auch ein Maß für dessen Konzentration. Das System mit Träger und Indikatorsubstanz wird im Folgenden als Indikator bezeichnet.

Vorrichtungen dieser Art sind seit längerem bekannt und werden beispielsweise auf dem Gebiet der Detektion von Analyten wie Explosivstoffe und andere gesundheits- und umweltrelevante Substanzen (Drogen, toxische Gase u.a.) eingesetzt. Dabei wird der Indikator unter definierten Bedingungen mit dem zu untersuchenden Medium kontaktiert, das z.B. gasförmig sein kann. Befinden sich im Medium z.B. Explosivstoffe, dann können diese die Emission des Indikators ändern.

In WO 03/031953 A2 ist eine optische Einheit dargestellt, bei der sich eine Indikatorsubstanz als Schicht auf der Strahlungsquelle für die Anregung der Emission befindet. Die Strahlungsgsquelle ist dabei eine GaN - LED. Diese Emission gelangt über einen optischen Filter auf einen Detektor, der die Emission in Vorwärtsrichtung erfasst.

In US 8287811 B1 wird eine optische Einheit vorgestellt, die einen Indikator mit zwei Indikatorsubstanzen auf einem gemeinsamen Träger enthält. Die beiden Indikatorsubstanzen sind auf den beiden, sich gegenüberliegenden Seiten des Trägers aufgebracht. Beide Indikatorsubstanzen werden mit derselben Wellenlänge im UV-Bereich bestrahlt. Wird die eine Indikatorsubstanz mit dem Analyten kontaktiert, so tritt eine Fluoreszenzlöschung ein. Die Fluoreszenz der anderen Indikatorsubstanz wird als Referenzsignal verwendet. Die Fluoreszenzen werden in Richtung einer Linse auf einen Photomultiplier gelenkt. Vor dem Photomultiplier ist ein Filterrad mit drei Filtern angeordnet.

US 8323576 B2 beschreibt ein Gerätesystem, dessen Herzstück ein zylindrischer Festkörper ist, der z.B. aus Glas bestehen kann. Dieser Körper ist als Kapillare ausgebildet und definiert beheizbar. Die Kapillare ist zweigeteilt. Der erste Teil dient als Gaseinlass und als Adsorptions - und Desorptionsstrecke für die zu detektierenden Analyten. Ein zweiter Teil enthält eine optische Einheit mit auf der Innenwand aufgebrachter Indikatorsubstanz. Eine Strahlungsquelle beaufschlagt über einen wellenlängenselektiven Filter den Indikator. Die Messung der Emission wird über Filter und Empfänger als Durchlichtmessung durchgeführt.

In WO 2012/134436 A1 wird ein Indikator beschrieben, der ebenfalls als Kapillare und gemeinsamer Träger für mehrere Indikatorsubstanzen ausgebildet ist. Neben der Fluoreszenz kommen auch Phosphoreszenz und Chemilumineszenz als Emissionssignale in Frage. In einem Beispiel enthält die Kapillare zwei Indikatorsubstanzen, die jeweils mit einer 405nm - LED bestrahlt werden. Die Messung der Emission erfolgt über Filter und Empfänger. Dabei dient der Indikator selbst als Lichtwellenleiter.

In WO 01/86263 A2 wird eine Vorrichtung vorgeschlagen, bei der die Anregungsstrahlung der Strahlungsquelle über einen Filter in einen Lichtwellenleiter, der als Verzweiger ausgebildet ist, einkoppelt und auf die Lichtwellenleiterendfläche geführt wird. Auf dieser Endfläche ist ein Polymer aufgebracht, das für eine Anreicherung des Analyten sorgt. Ein Teil der Emission wird von demselben Lichtwellenleiter zum Verzweiger zurückgeführt und auf einen Detektor mit Filter gelenkt. Die Vorrichtung gestattet die Aufnahme mehrerer solcher Lichtwellenleiteranordnungen. Das Gesamtsystem kann damit verschiedene Anregungsquellen, Filter, Polymere und Detektoren aufweisen.

In US 2014/0065720 A1 wird die Fluoreszenz über die Surface-Plasmon-Coupled-Emission Methode (SPCE) erzeugt. Die Fluoreszenz wird mit einem hohen Signal-Rausch-Verhältnis erfasst, so dass diese mit hoher spektraler Auflösung gemessen werden kann. Der Indikator enthält zur Durchführung der SPCE zusätzlich eine Metallschicht und eine dielektrische Schicht. Der Sensor wird mit einer Strahlungsquelle für Anregungsstrahlung dargestellt.

In WO 2008/051189 A2 wird ein Fluoreszenzsystem vorgestellt, das sowohl anregungsseitig als auch emissionsseitig zum Zweck der spektralen Auflösung mit Filterrädern ausgestattet ist. Mit einem solchen System stehen viele Anregungs- und Emissionswellenlängen zur Verfügung.

US2004/106211 A1 offenbart die Merkmale des Oberbegriffs des Anspruchs 1.

Die Geräte sollen für die Detektion von Analyten wie Explosivstoffe und andere gesundheits- und umweltrelevante Substanzen geeignet sein. Sie sind zu einem Teil mit einer hohen technischen Funktionalität ausgestattet und entsprechend komplex aufgebaut. Zu einem anderen Teil enthalten die Geräte eine minimalisierte technische Ausstattung.

### AUFGABE UND LÖSUNG

Es ist eine Aufgabe der Erfindung, ein feldtaugliches, portables Gerät mit einer Vorrichtung zur Erzeugung und Messung einer Emission für den Einsatz bei der Detektion von Explosivstoffen bereitzustellen, das gut an die Applikation in rauen Umgebungen angepasst ist, von einem Bediener gut gehandhabt werden kann, einen relativ einfachen, robusten Aufbau hat und dennoch durch eine hohe Messsicherheit charakterisiert ist.

Diese Aufgabe wird durch ein portables Gerät mit den Merkmalen von Anspruch 1 gelöst. Vorteilhafte Weiterbildungen sind in den abhängigen Ansprüchen angegeben. Der Wortlaut sämtlicher Ansprüche wird durch Bezugnahme zum Inhalt der Beschreibung gemacht.

Das portable Gerät ist aufgrund seiner Konstruktion zur Detektion von Explosivstoffen mittels der Messung von optischen Emissionen geeignet. Dazu werden entsprechende, auf diese Substanzen empfindlich reagierende Indikatoren verwendet, deren Veränderung durch Anwesenheit der gesuchten Analyten optisch detektierbar ist. Es können ggf. auch andere gesundheits- und umweltrelevante Substanzen (wie z.B. Drogen, toxische Gase u.a.) detektiert werden. Dazu können eventuell andere Indikatoren verwendet werden. Die Vorrichtung zur Erzeugung und Messung von Emission eines Indikators ist ein wesentlicher funktioneller Bestandteil des Geräts.

Die Vorrichtung zur Erzeugung und Messung von Emission weist mehrere Strahlungsquellen auf, die jeweils quasi-monochromatische Anregungsstrahlung aussenden können. Der Begriff "quasi-monochromatische Anregungsstrahlung" bezeichnet in dieser Anmeldung Anregungsstrahlung aus einem relativ engen Wellenlängenbereich, beispielsweise mit einer spektralen Bandbreite von deutlich weniger als 100 nm, wobei die spektrale Bandbreite z.B. in der Größenordnung von ca. 10 nm oder wenigen 10 nm liegen kann. Die Anregungsstrahlung kann auch als "schmalbandige Anregungsstrahlung" bezeichnet werden. Der Begriff "Anregungswellenlänge" bezieht sich dann auf einen relativ engen Wellenlängenbereich. Bei der Anregungsstrahlung handelt es sich somit insbesondere nicht um breitbandiges Weißlicht. Als Strahlungsquellen zur Erzeugung der Anregungsstrahlung können z.B. Lichtemittierende Dioden (LED) oder Laserdioden verwendet werden oder breitbandige Weißlichtquellen, deren Strahlung über mindestens eine wellenlängenselektive Einrichtung (z.B. ein Gitter oder ein schmalbandiges Filter) geführt ist.

Die Anregung erfolgt somit nicht mit einem kontinuierlichen breitbandigen Spektrum (z.B. mittels Weißlicht), sondern in einem einzigen schmalbandigen Wellenlängenbereich oder mehreren spektral unterschiedlichen, relativ schmalbandigen Wellenlängenbereichen.

Die Strahlungsquellen sind in einer definierten räumlichen Anordnung mit gegenseitigem Abstand zueinander angeordnet. Die räumliche Verteilung der Strahlungsquellen in der Strahlungsfläche bestimmt das vom Ort abhängige Muster von Strahlungsquellen, welches für die Messung genutzt werden kann.

Das Anregungslicht gelangt über einen Anregungsstrahlengang auf die Indikatorfläche. Der Anregungsstrahlengang weist hierzu mindestens ein erstes Abbildungssystem auf. Emissionen der Indikatorsubstanzen können dann an den Orten erzeugt werden, an denen die Anregungsstrahlung über den Anregungsstrahlengang auf der Indikatorfläche oder auf die Indikatorfläche abgebildet wird. Diese Orte bilden dann ein Muster in Abhängigkeit vom Ort in der Indikatorfläche. Das Muster dieser Orte kann dem Muster der Strahlungsquellen im Sinne einer geometrischen Ähnlichkeit entsprechen, dies ist jedoch nicht zwingend. In der Indikatorfläche können mehrere lateral verteilte Orte mit Anregungsstrahlung beaufschlagt werden. Die örtliche Verteilung der Anregungsstrahlung auf der Indikatorfläche und die örtliche Verteilung der Indikatorsubstanzen sind aufeinander abgestimmt.

Ein Emissionsstrahlengang dient zur Abbildung der Indikatorfläche in eine Empfangsfläche derart, dass in der Empfangsfläche ein Muster von Emissionen in Abhängigkeit vom Ort erzeugbar ist. Emissionen können also an unterschiedlichen Orten der Empfangsfläche auftreffen. Der Emissionsstrahlengang weist hierzu mindestens ein zweites Abbildungssystem auf. Das Muster von Emissionen in der Empfangsfläche kann dem Muster von mit Anregungsstrahlung beaufschlagten Orten der Indikatorfläche im Sinne einer geometrischen Ähnlichkeit entsprechen, dies ist jedoch nicht zwingend.

Wenn im Rahmen dieser Anmeldung von einem "Abbildungssystem" oder einer abbildenden Optik die Rede ist, so soll dies bedeuten, dass das Abbildungssystem von einem Gegenstand eine vergrößerte, verkleinerte oder eine 1:1-Abbildung erzeugen kann, ohne dabei die Form bzw. das Aussehen des Gegenstandes (abgesehen von Abbildungsfehlern) zu verändern. Diejenige Fläche oder Ebene, auf der oder die abgebildet wird, kann dabei in einer Bildebene des Abbildungssystems oder außerhalb der Bildebene liegen.

Es sind mehrere Empfänger für den Empfang von Emissionen aus der Empfangsfläche und zur Wandlung der empfangenen Emissionen in elektrische Signale vorgesehen. Dabei ist ein Empfänger höchstens einer Indikatorsubstanz zugeordnet in dem Sinne, dass es zu einem gegebenen Zeitpunkt keine zwei oder mehr Indikatorsubstanzen gibt, denen derselbe Empfänger zugeordnet ist. Ein Empfänger empfängt somit zu einem gegebenen Zeitpunkt Emissionsstrahlung maximal von genau einer ihm zugeordneten Indikatorsubstanz. Die Emissionen in der Eintrittsfläche sind örtlich derart voneinander entkoppelt, dass die Emissionen ohne gegenseitige Überlagerung von Emissionen auflösbar sind. Die von einem Empfänger empfangene Emission kann genau einer der Indikatorsubstanzen und einer der Strahlungsquellen zugeordnet werden. Hierdurch sind hochselektive Messungen mit hoher Messgenauigkeit möglich. Ein Empfänger kann optimal auf nur die eine ihm zugeordnete Indikatorsubstanz optisch justiert werden. Dadurch wird eine optimale Abstimmung der Fläche (Flächenausdehnung, -form und -größe der Fläche) der Indikatorsubstanz auf die aktive Fläche des Empfängers möglich. Dadurch kann ein hohes Signal-Rausch-Verhältnis (S/N Verhältnis, signal to noise ratio) erzielt werden.

Das Gerät ist als portables, von einer einzigen Person leicht tragbares und bedienbares Gerät ausgelegt. Das Gerät kann insbesondere so klein und leicht konstruiert sein, dass es bei der Benutzung mit nur einer Hand gehalten werden kann (Handheld-Gerät). Baugröße und Gewicht sowie die Robustheit der darin verbauten Komponenten sind im Hinblick auf diese Anwendung optimiert. Das Gewicht kann z.B. weniger als 2 Kilogramm betragen, insbesondere weniger als 1.5 Kilogramm, meist jedoch mindestens 0.5 Kilogramm. Die Baugröße inklusive Halteeinrichtung für den Indikator kann z.B. bei 50 cm oder weniger, oder bei 40 cm oder weniger, oder bei 30 cm oder weniger (gemessen in der Richtung größter Ausdehnung) liegen, wobei die Baugröße meist jedoch bei mindestens 10 cm bis 15 cm liegt.

Obwohl es prinzipiell möglich ist, den Anregungsstrahlengang und den Emissionsstrahlengang auf unterschiedlichen Seiten der Indikatorfläche anzuordnen, ist erfindungsgemäß vorgesehen, dass der Anregungsstrahlengang und der Emissionsstrahlengang in Rückwärtsgeometrie angeordnet sind, so dass der Anregungsstrahlengang und der Emissionsstrahlengang auf ein und derselben Seite der Indikatorfläche bzw. des Indikators angeordnet sind. Auf diese Weise ist es besonders einfach möglich, den Indikator in einer Haltevorrichtung unterzubringen, die an eine Seite des Geräts angekoppelt und bei Bedarf leicht ausgewechselt werden kann. Die Rückwärtsgeometrie kann auch bei einer in das Gerät fest integrierten Haltevorrichtung günstig sein, da z.B. alle optischen Komponenten auf der gleichen Seite der Indikatorfläche untergebracht werden können.

Die Messgeometrie ist durch die Konstruktion so eingestellt, dass vom Indikator spekular reflektierte Anregungsstrahlung und damit für die Emissionsmessung störende Strahlung nicht in relevantem Ausmaß auf die Empfangsfläche trifft und damit auch nicht zu einem Empfänger gelangen und dort den Störhintergrund erhöhen kann. Bei bevorzugten Ausführungsformen sind zur Vermeidung oder Verminderung des Eintritts von spekular reflektierten Intensitätsanteilen der Anregungsstrahlung in den Emissionsstrahlengang unterschiedliche Winkel von Anregungsstrahlung und Emissionsstrahlung zur Oberflächennormalen des Indikators im Bereich des Auftreffens der Anregungsstrahlung vorgesehen.

Das wird vorzugsweise dadurch erreicht, dass optische Achsen von einem oder mehreren oder allen Abbildungssystemen des Anregungsstrahlengangs (erstes Abbildungssystem) und des Emissionsstrahlengangs (zweites Abbildungssystem) im Bereich der Indikatorfläche schräg zueinander verlaufen und nicht symmetrisch zur Oberflächennormalen angeordnet sind. Ein Winkel zwischen einfallender Anregungsstrahlung und emittierter Emissionsstrahlung kann z.B. im Bereich von 20° bis 60° liegen, insbesondere im Bereich von 30° bis 50°.

Zur Separierung von Anregungsstrahlengang und Emissionsstrahlengang ist vorzugsweise kein physikalischer Strahlteiler (wie z.B. ein dichroitischer Spiegel oder Polarisationsstrahlteiler) oder geometrischer Strahlteiler (wie z.B. ein Spiegel mit mindestens einer Durchbrechung im ausgeleuchteten Bereich) erforderlich und auch nicht vorgesehen. Typische bei der Verwendung von Strahlteilern mögliche Probleme, z.B. durch am Strahlteiler generiertes Streulicht, können dadurch vermieden werden.

Die Anordnung von Anregungsstrahlengang und Emissionsstrahlengang kann so getroffen sein, dass optische Achsen von den Abbildungssystemen des Anregungsstrahlengangs (erstes Abbildungssystem bzw. erste Abbildungssysteme) und einem oder mehreren oder allen Abbildungssystemen des Emissionsstrahlengangs (zweites Abbildungssystem bzw. zweite Abbildungssysteme) sich mit Abstand vor der Indikatorfläche kreuzen, so dass Durchstoßpunkte der optischen Achsen in der Indikatorfläche lateral auseinanderliegen. Dadurch kann sichergestellt werden, dass Anregungsstrahlung, die an einer oder mehreren mindestens partiell reflektierenden Grenzflächen des Indikators (z.B. Grenzfläche Luft-Indikator) spekular reflektiert wird, praktisch nicht in den Emissionsstrahlengang und damit nicht bis zu den Empfängern gelangen kann. Das trägt dazu bei, dass der Gebrauch von Anregungsfiltern und/oder Emissionsfiltern gegebenenfalls entfallen kann. Der Abstand liegt deutlich außerhalb der Fertigungstoleranzen und kann z.B. im Bereich von 1 mm bis 10 mm liegen, ggf. auch darunter oder darüber.

Manche Ausführungsformen sind gekennzeichnet durch eine wellenlängenselektive Einrichtung mit einer Eintrittsfläche und einer Austrittsfläche, wobei die Eintrittsfläche mit der in der Empfangsfläche empfangenen Emission beaufschlagbar ist und in der Austrittsfläche die Empfänger angeordnet sind. Damit können bestimmte engere Wellenlängenbereiche aus einem breiteren Emissionsspektrum selektiert bzw. für die Auswertung ausgewählt werden. Es kann für alle Empfänger der gleiche Wellenlängenbereich ausgewählt werden. Es ist auch möglich, für unterschiedliche Empfänger unterschiedliche, mit spektralem Abstand zueinander liegende Wellenlängenbereiche auszuwählen. Die wellenlängenselektive Einrichtung kann auswechselbar sein, um das Gerät an unterschiedliche Messaufgaben optimal anpassen zu können. Eine wellenlängenselektive Einrichtung zwischen der Empfangsfläche und den Empfängern ist jedoch nicht zwingend nötig.

Es kann jeder Indikatorsubstanz ein separater Empfänger zugeordnet sein. Zwischen den Empfängern können Zwischenräume ohne lichtempfindliche Flächen liegen. Die Empfänger können individuell bzw. separat voneinander justierbar sein, so dass jeder Messkanal für seine Messaufgabe optimiert eingestellt werden kann. Die Anzahl der Empfänger kann der Anzahl der effektiv nutzbaren Anregungskanäle entsprechen.

Vorzugsweise ist vorgesehen, dass jeder Indikatorsubstanz eine oder mehrere Strahlungsquellen zugeordnet sind, wobei eine Strahlungsquelle höchstens einer Indikatorsubstanz zugeordnet ist, so dass es keine zwei oder mehr Indikatorsubstanzen gibt, denen dieselbe Strahlungsquelle zugeordnet ist. Die Strahlungsquelle kann dadurch optimal auf nur die eine, ihr zugeordnete Indikatorsubstanz optisch justiert werden. Dadurch ist eine optimale Abstimmung der Fläche der Indikatorsubstanz auf diejenige der Strahlungsquelle möglich. Dies ist förderlich zur Erzielung eines hohen Signal-Rausch-Verhältnisses.

Bei vielen Ausführungsformen werden als Empfänger Photomultiplier verwendet, die hohe Empfindlichkeit selbst bei geringen messrelevanten Intensitäten bieten. Insbesondere können halbleiterbasierte Photomultiplier verwendet werden, z.B. Si-Photomultiplier (Si-PMT). Diese sind mechanisch robust, relativ unempfindlich gegen Erschütterungen und bieten sehr hohe Empfindlichkeiten bereits bei relativ kleinen sensitiven Flächen. Alternativ können z.B. konventionelle Photomultiplier oder Photodioden als Empfänger verwendet werden.

Wie erwähnt kann ein Abbildungssystem von einem Gegenstand eine vergrößerte, verkleinerte oder eine 1:1-Abbildung erzeugen, ohne dabei die Form bzw. das Aussehen des Gegenstandes (abgesehen von Abbildungsfehlern) zu verändern. Diejenige Fläche oder Ebene, auf der oder die abgebildet wird, kann dabei in einer Bildebene des Abbildungssystems oder außerhalb der Bildebene liegen. Wenn von einer "nicht abbildenden Optik" die Rede ist, so soll dies bedeuten, dass die verwendeten optischen Elemente (eines oder mehrere) als Strahlformer fungieren, die die Form bzw. das Aussehen eines Gegenstandes ändern können. In einem optischen Kanal (Anregungskanal oder Emissionskanal) kann abbildende Optik und nicht abbildende Optik kombiniert sein.

Außerhalb der Erfindung ist es möglich, die Vorrichtung so aufzubauen, dass der Anregungsstrahlengang nur einen einzigen Anregungskanal mit einem ersten Abbildungssystem aufweist, dem dann zwei oder mehr Strahlungsquellen zugeordnet sind. Der Anregungskanal kann mehrere Sub-Kanäle für die von den Strahlungsquellen zur Indikatorfläche laufende Anregungsstrahlung bei einer gemeinsamen optischen Achse bereitstellen.

Die Vorrichtungen der Erfindung sind dagegen so konstruiert, dass der Anregungsstrahlengang mehrere, das heißt zwei oder mehr, Anregungskanäle aufweist, wobei jeder Anregungskanal ein erstes Abbildungssystem aufweist, das eine optische Achse des entsprechenden Anregungskanals definiert. Die Anzahl der voneinander separaten Anregungskanäle kann beispielsweise im Bereich von zwei bis zehn liegen, insbesondere im Bereich von drei bis sechs. Damit kann die Anregungsstrahlung unterschiedlicher Strahlungsquellen auf separaten, optisch voneinander getrennten Anregungskanälen zum Indikator geführt werden. Ein Übersprechen (cross talk) zwischen den Anregungskanälen wird dadurch konstruktiv verhindert.

Der Begriff "Anregungskanal" bezeichnet in dieser Anmeldung ein optisches System mit mindestens einem mit Brechkraft ausgestalteten optischen Element, beispielsweise einer Linse, und gegebenenfalls weiteren optischen Elementen, wie beispielsweise mindestens einem Strahlformer und/oder mindestens einem Filter. Ein Anregungskanal führt Strahlung von einer oder mehreren Strahlungsquellen in definierter Weise in die Indikatorfläche.

Die Anordnung kann so getroffen sein, dass für jeden Indikatorbereich genau ein Anregungskanal vorgesehen ist, so dass die Anzahl der Anregungskanäle der Anzahl der Indikatorbereiche entspricht. Der Begriff "Indikatorbereich" bezeichnet den Ort der Indikatorsubstanz bzw. den Bereich, in dem sich eine Indikatorsubstanz befindet. Es ist auch möglich, einen Anregungskanal zur Leitung von Anregungsstrahlung auf zwei oder mehr voneinander separierte Indikatorbereiche zu nutzen.

Für den Emissionsstrahlengang können ebenfalls mehrere voneinander gesonderte Emissionskanäle vorgesehen sein.

Bei einer Ausführungsform ist dagegen vorgesehen, dass der Emissionsstrahlengang für alle Emissionen ein gemeinsames Abbildungssystem (zweites Abbildungssystem) aufweist, das eine optische Achse des Emissionsstrahlengangs definiert. Es kann also ein einziger Emissionskanal ausreichen. Dieser kann mehrere Sub-Kanäle für die vom Indikator zur Empfangsebene laufenden Emissionen bei einer gemeinsamen optischen Achse bereitstellen.

Bei einer Ausführungsform mit mehreren Anregungskanälen bilden die Anregungskanäle in Bezug auf den Emissionskanal eine symmetrische Anordnung. Insbesondere kann die Anordnung eine Spiegelsymmetrie zu mindestens einer die optische Achse des Emissionsstrahlengangs enthaltenden Symmetrieebene aufweisen.

Bei einer Ausführungsform mit mehreren Anregungskanälen bilden die Anregungskanäle und der Emissionskanal eine kegelförmige Anordnung, wobei optische Achsen der Anregungskanäle auf einer Kegelmantelfläche angeordnet sind, die den Emissionskanal umgibt, und die optische Achse des Emissionskanals in der Achse des Kegels angeordnet ist. Hierdurch können in sehr kompakten Abmessungen eine Vielzahl von strahlungsführenden Kanälen zwischen den Strahlungsquellen, dem Indikator und der Empfangsebene bzw. den Empfängern bereitgestellt werden. Die Kegelspitze der kegelförmigen Anordnung, also der Schnittpunkt der optischen Achsen der Anregungskanäle, liegt bei bevorzugten Ausführungsformen nicht in der Indikatorfläche, sondern mit Abstand von dieser entfernt, insbesondere vor der Indikatorfläche.

In manchen Fällen kann es zweckmäßig sein, wenn in einem Anregungskanal zwischen einer Strahlungsquelle und der Indikatorebene ein Strahlformer angeordnet ist. Der Begriff "Strahlformer" bezeichnet hier allgemein eine nichtabbildende Optik, die die Form bzw. das Aussehen eines Gegenstandes ändern kann. Ein Strahlformer kann z.B. eine Diffusorfunktion haben. Der Strahlformer kann z.B. ein mikrostrukturiertes Bauelement (z.B. ein diffraktives optisches Element) aufweisen und/oder dafür ausgelegt sein, ein ungleichmäßiges Intensitätsprofil in ein gleichmäßiges und steiles Intensitätsprofil umzuwandeln. Hiermit kann auch bei Verwendung von kleinen, quasi punktförmigen Strahlungsquellen mit ungleichmäßiger Abstrahlcharakteristik eine gleichmäßige bzw. homogene Ausleuchtung von anzuregenden Bereichen (Spots) der Indikatorsubstanzen erreicht werden, wodurch aussagekräftige quantitative Vergleiche ermöglicht und die Messgenauigkeit gesteigert werden kann.

Bei manchen Ausführungsformen ist ein Taktgeber zum Ansteuern von Strahlungsquellen vorgesehen, wobei der Taktgeber derart konfiguriert ist, dass alle Strahlungsquellen seriell nacheinander oder dass ein Teil der Strahlungsquellen oder alle Strahlungsquellen gleichzeitig getaktet wird/werden oder dass Strahlungsquellen abwechselnd getaktet werden. Die Strahlungsquellen können dadurch im Blitzbetrieb arbeiten, so dass sie zeitlich in definierter Weise mit einem Taktgenerator ein- und ausgeschaltet werden können. Diese Varianten können so beschrieben werden, dass die Strahlungsfläche nicht nur ein Muster von Strahlungsquellen in Abhängigkeit vom Ort darstellt, sondern auch ein Muster in Abhängigkeit von der Zeit. In diesem Fall kann ein Muster immer dann vorliegen, wenn mindestens eine Abhängigkeit (Ort, Zeit) besteht.

Mithilfe eines Taktgebers ist es auch möglich, in einem Anregungskanal eine sekundäre oder virtuelle Strahlungsquelle zu erzeugen, die unterschiedliche Anregungswellenlängen wahlweise abgeben kann. Bei manchen Ausführungsformen sind mindestens einem der Anregungskanäle zwei oder mehr Strahlungsquellen zur Abgabe unterschiedlicher Anregungswellenlängen zugeordnet, wobei die Strahlungsquellen mittels eines Taktgebers wechselweise taktbar sind und eine Strahlungsvereinigungseinrichtung zur wahlweisen Einkopplung von Anregungsstrahlung der Strahlungsquellen in den Anregungskanal vorgesehen ist. Die Strahlungsvereinigungseinrichtung kann beispielsweise einen Strahlformer in Form eines Diffusors aufweisen.

Eine besonders kompakte und robuste Ausgestaltung wird bei manchen Ausführungsformen dadurch erreicht, dass alle optischen Elemente der Vorrichtung, das heißt alle optischen Elemente des Anregungsstrahlengangs und des Emissionsstrahlengangs, in einem einzigen Bauteil eingebaut bzw. integriert sind. Das Bauteil kann als Optikhalter für alle optischen Elemente dienen. Es kann aus einem einzigen Stück bestehen (monolithisches Bauteil) oder aber aus zwei, drei oder mehr Komponenten fest zusammengefügt werden. Ein solches Bauteil kann beispielsweise als Spritzgussteil hergestellt werden, oder durch Sintern oder durch materialabtragende Bearbeitung aus dem Vollen. Als optische Elemente werden alle Komponenten in der Vorrichtung angesehen, die die Eigenschaften von Strahlung räumlich, zeitlich und spektral verändern können. Das sind vornehmlich Funktionen der Abbildung, Lenkung und Leitung sowie Formung der Strahlung. Durch die gemeinsame Unterbringung aller optischen Elemente in ein und demselben als Optikhalter dienenden Bauteil bleibt die relative Anordnung der optischen Elemente zueinander auch bei stärkeren Bewegungen der gesamten Vorrichtung, bei Stößen oder Erschütterungen durchgängig erhalten. Das Gerät kann also auch in rauen Umgebungen eingesetzt werden, Messungen sind selbst dann mit hoher Genauigkeit möglich, wenn ein Bediener das Gerät mit Hand hält und das gesamte Gerät dadurch leicht bewegt wird.

Vorzugsweise sind in dem Bauteil mehrere Aufnahmekanäle zur Aufnahme der optischen Elemente von mindestens einem Anregungskanal und einem Emissionskanal ausgebildet. Die Aufnahmekanäle, die z.B. durch Bohren oder Erodieren in ein zunächst massives Bauteil eingebracht werden können, können z.B. jeweils rotationssymmetrische Gestalt haben, andere Querschnittsformen sind möglich. Vorzugsweise sind die optischen Elemente einzeln oder in Gruppen in z.B. hülsenförmigen Fassungselementen gefasst, die in zugeordnete Abschnitte der Aufnahmekanäle im Bauteil passen und beim Zusammenbau der Vorrichtung in die Aufnahmekanäle einführbar und dort fixierbar sind.

Ein Gerät, das spezifisch zur Detektion von Explosivstoffen konfiguriert ist, hat vorzugsweise eine an die Empfänger angeschlossene Auswerteeinrichtung zum Empfang von elektrischen Signalen der Empfänger und zur Auswertung der in den Signalen enthaltenen spektralen Information über die Emissionen, wobei die Auswerteeinrichtung so konfiguriert ist, dass mindestens eine der folgenden Informationen ermittelbar und an einen Bediener oder Nutzer der Information ausgebbar ist: Informationen über die Menge eines oder mehrerer gesuchter Analyten; Information über die Art einer oder mehrerer Analyten. Dabei umfassen die Analyten jeweils einen oder mehrere Explosivstoffe oder Gruppen von Explosivstoffen. In einem Speicher der Auswerteeinrichtung kann Information über spektrale Charakteristika von Fluoreszenzspektren und/oder Lumineszenzspektren der Zielsubstanzen in Form von Zielsubstanz-Daten gespeichert sein, auf die bei der Auswertung für Vergleichsoperationen zurückgegriffen werden kann.

Die Erfindung betrifft auch die Verwendung des Geräts zur Detektion von Explosivstoffen, wobei ein Indikator verwendet wird, der mindestens eine Indikatorsubstanz aufweist, die bei Bestrahlung mit Anregungsstrahlung und Kontakt mit einem mindestens einen Explosivstoff enthaltenden Analyten eine Verminderung oder Verstärkung der von der Indikatorsubstanz abgegebenen Fluoreszenzintensität zeigt.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind.
Fig. 1 zeigt schematisch ein Ausführungsbeispiel eines portablen Geräts zur Detektion von Explosivstoffen mithilfe von Fluoreszenzmessungen;
Fig. 2 zeigt in schrägperspektivischer Darstellung Komponenten einer optischen Vorrichtung, die in richtiger räumlicher Anordnung zusammen mit einer angekoppelten Haltevorrichtung für einen Indikator dargestellt sind;
Fig. 3 zeigt einen Schnitt durch Komponenten einer optischen Vorrichtung, die in richtiger räumlicher Anordnung zusammen mit einer angekoppelten Haltevorrichtung für einen Indikator dargestellt ist;
Fig. 4 zeigt Verläufe von Strahlen in einem Anregungskanal des Anregungsstrahlengangs und im Emissionsstrahlengang, wobei am Indikator spektral reflektierte Strahlung nicht in den Emissionsstrahlengang fällt;
Fig. 5 zeigt schematisch einige Komponenten einer Vorrichtung zur Erzeugung und Detektion optischer Emissionen von Indikatoren gemäß einer Ausführungsform mit nur einem Anregungskanal und einem Emissionskanal;
Fig. 6 bis 12 zeigen schematisch räumliche Verteilungen bzw. Muster von Strahlungsquellen, bestrahlten Flächen und Emissionen in verschiedenen aufeinanderfolgenden ausgezeichneten Flächen entlang der Strahlengänge unterschiedlicher Beispiele.

### DETAILLIERTE BESCHREIBUNG DER AUSFÜHRUNGSBEISPIELE

Bei der folgenden Darstellung von Ausführungsbeispielen werden gelegentlich strukturell und/oder funktional gleiche oder einander entsprechende Komponenten aus Gründen der Übersichtlichkeit mit gleichen Bezugszeichen gekennzeichnet, auch wenn sie zu unterschiedlichen Ausführungsbeispielen gehören oder dort verwendet werden können.

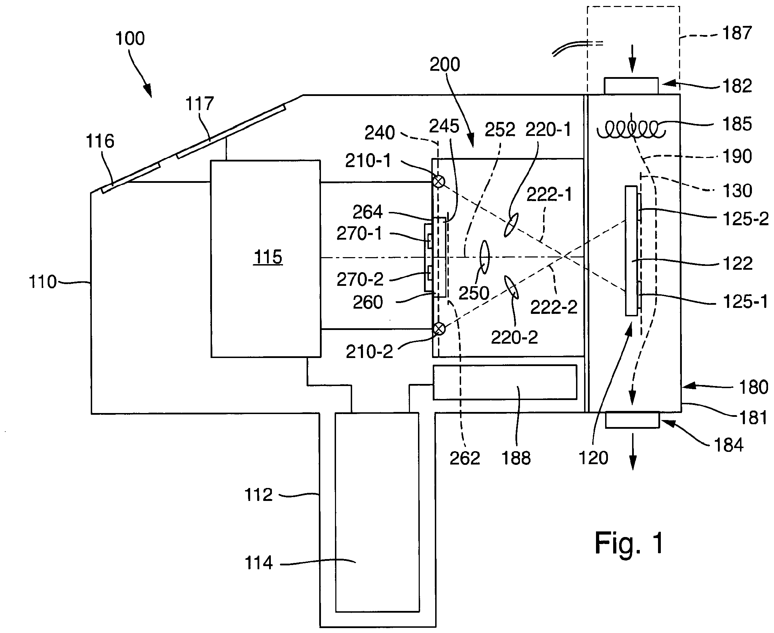

In Fig. 1 ist schematisch ein Ausführungsbeispiel eines Geräts 100 zur Detektion von Explosivstoffen dargestellt. Das Gerät nutzt für diesen Zweck u.a. Fluoreszenzmessungen, also optische Verfahren.

Es handelt sich um ein portables Gerät, welches insbesondere mit nur einer Hand eines Bedieners gehalten und herumgetragen werden kann (Handheld-Gerät). Bevorzugte Anwendungsgebiete liegen z.B. im Bereich der Gepäckkontrolle, der Fahrzeugkontrolle, der Personenkontrolle und/oder der Gebäudekontrolle auf Explosivstoffe. Das Gerät ist innerhalb und außerhalb von Gebäuden einsetzbar, z.B. bei zeitlich befristeten Einzelkontrollen und/oder Spot Checks. Es kann in vielen Fällen als Ersatz für den Einsatz von Spürhunden dienen.

Das Gerät hat ein stabiles, lichtdichtes Gehäuse 110, das z.B. aus Metall, einem schlagfesten Kunststoff oder einer Kombination dieser Materialien bestehen kann. Das Gehäuse dient der Handhabung des Geräts sowie der Halterung und dem Schutz der daran und darin untergebrachten Komponenten.

An der Unterseite des Gehäuses 110 des Geräts ist ein Handgriffabschnitt 112 angebracht oder ausgebildet. Dieser dient zur einhändigen Handhabung des Geräts. Innerhalb des Handgriffabschnitts 112 kann beispielsweise ein Batteriepaket oder Akkumulatorpaket oder eine andere netzunabhängige elektrische Leistungsversorgung 114 untergebracht sein. Eine Steuereinheit 115 mit einem oder mehreren Mikrocontrollern enthält alle zur Steuerung der Funktionen des Geräts erforderlichen elektrischen und elektronischen Komponenten. In der Steuereinheit ist auch eine Auswerteeinrichtung integriert. Die Bedienung des Geräts erfolgt über ein an der Gehäuseoberseite angebrachtes Bedienfeld 116. Über eine Anzeigeeinrichtung 117 kann Bedienerinformation angezeigt werden. Es ist möglich, das Bedienfeld und die Anzeigeeinrichtung durch einen berührungsempfindlichen Bildschirm (Touchscreen) zu realisieren.

Im vorderen Teil des Gehäuses 110 ist eine Vorrichtung 200 zur Erzeugung und Messung von Emissionen eines Indikators 120 angeordnet. Der Indikator weist einen Träger 122 auf, der im Beispielsfall durch eine planparallele Platte aus Glas oder einem für die genutzte Strahlung (sichtbares Spektrum und nahes UV-Spektrum) transparenten Kunststoff besteht. Der Träger kann, wie im Beispielsfall, einteilig (monolithisch) sein oder aus mehreren Komponenten zusammengesetzt sein.

An der der Vorrichtung 200 abgewandten Seite (Rückseite) des Trägers sind mehrere Indikatorsubstanzen 125-1, 125-2, etc. jeweils als dünne Schicht auf dem Träger 122 aufgebracht. Die Indikatorsubstanzen können unmittelbar auf eine Fläche des Trägers aufgebracht sein. Es ist auch möglich, eine transparente Zwischenschicht vorzusehen, die dann als Teil des Trägers angesehen werden kann.

Die Indikatorsubstanzen können mit einem fluiden, insbesondere gasförmigen Analyten 190 beaufschlagt bzw. mit diesem in Kontakt gebracht werden.

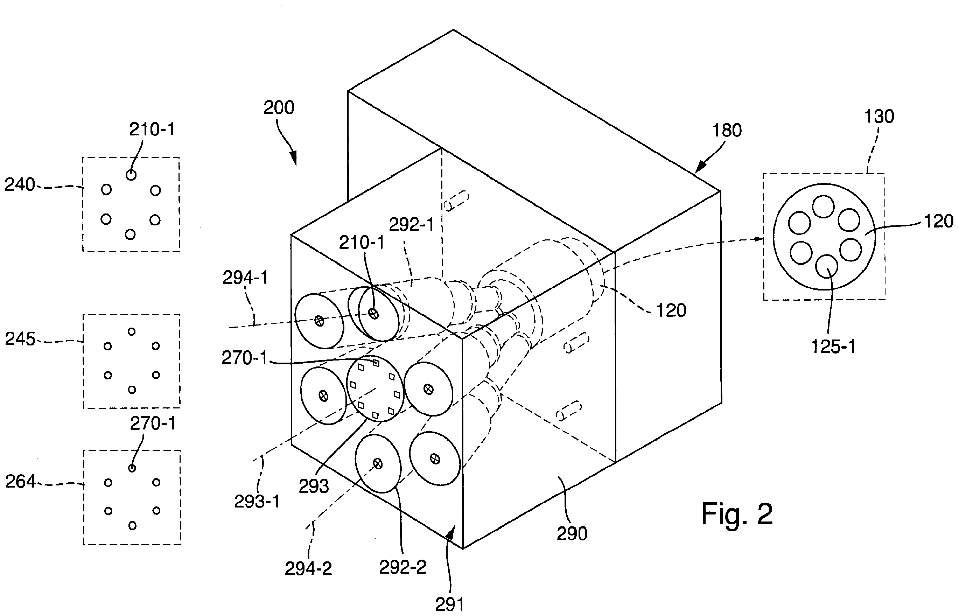

Der Indikator ist in einer Haltevorrichtung 180 untergebracht und dort in präzise definierter Position gehalten. Die Haltevorrichtung 180 selbst ist auswechselbar am Gehäuse 110 des Geräts 100 angebracht. Bei anderen Ausführungsformen ist eine Haltevorrichtung in das Gerät integriert und selbst nicht auswechselbar, jedoch ist der Indikator auswechselbar. Im dargestellten betriebsfertig montierten Zustand des Geräts (mit angekoppelter Haltevorrichtung 180 und darin untergebrachtem Indikator 120) sind die Indikatorsubstanzen 125-1, 125-2 präzise in einer Indikatorfläche 130 positioniert, bei der es sich im Beispielsfall um eine ebene Fläche handelt, die auch als Indikatorebene bezeichnet werden kann. Wie schon die schematische Fig. 1 zeigt, sind die Indikatorsubstanzen 125-1, 125-2 in unterschiedlichen, das heißt räumlich voneinander getrennten Bereichen des Trägers 122 derart angeordnet, dass die Indikatorsubstanzen in der Indikatorfläche 130 ein Muster von Indikatorsubstanzen in Abhängigkeit vom Ort darstellen. Die jeweils räumlich begrenzten, relativ kleinflächig aufgebrachten Indikatorsubstanzen sind also in wohl definierten Positionen innerhalb der Indikatorfläche 130 positioniert. Die Indikatorsubstanzen können z.B. in Form von kreisrunden kleinflächigen Beschichtungen mit einigen Millimetern Durchmesser auf einen Kreis um ein Zentrum angeordnet sein (vgl. Fig. 2). Sie können unmittelbar oder unter Zwischenschaltung einer transparenten Zwischenschicht auf den Träger aufgebracht sein.

Die Haltevorrichtung 180 hat ein eigenes Gehäuse 181 und ist vom Gehäuse 110 abnehmbar und in präzise vorgebbarer Position ankoppelbar. Die Haltevorrichtung 180 weist einen Zuluftanschluss 182 mit einer Einlassöffnung zum Ansaugen gasförmiger Medien auf, die den oder die gesuchten Analyten tragen. Mithilfe einer Heizeinrichtung 185 kann das angesaugte Medium vor Kontakt mit den Indikatorsubstanzen auf geeignete Temperaturen aufgeheizt werden. Das gegebenenfalls temperierte Medium mit den Analyten wird dann mithilfe eines geeigneten, vorzugsweise beheizbaren, Fluidkanalsystems über die Indikatorsubstanzen geführt und nach Kontakt mit diesen über einen Abluftanschluss 184 aus der Haltevorrichtung 180 ausgegeben.

An die Zuluftseite der Haltevorrichtung 180 kann ein mit einer eigenen Heizung ausgestattetes Wischprobenmodul 187 angekoppelt werden, mit welchem Analyten, die über Wischproben aufgenommen wurden, der Zuluft zugeführt werden. Die Strömung des Analyten durch das Fluidkanalsystem der Haltevorrichtung wird mittels einer elektrischen Pumpe 188 erzeugt, die an die Leistungsversorgung 114 angeschlossen und im Gehäuse 110 untergebracht ist.

In der in Fig. 1 gezeigten Längsrichtung (von links nach rechts) ist das Gerät inklusive der daran angebrachten Haltevorrichtung 180 für den Indikator 120 nicht länger als 40 Zentimeter. Das Gerät wiegt weniger als 1.5 Kilogramm. Es kann daher bequem einhändig gehandhabt werden.

Die im Gehäuse 110 untergebrachte Vorrichtung 200 ist als eine kompakte optische bzw. optoelektronische Einheit ausgeführt, mit der Emissionen des Indikators 120 bzw. der Indikatorsubstanzen 125-1 etc. erzeugt und diese Emissionen mithilfe optischer und optoelektronischer Einrichtungen gemessen werden können. Der Begriff "Emission" ist hier im Sinne einer optischen Emission zu verstehen. Bei der Emission handelt es sich also um elektromagnetische Strahlung, die von den Indikatorsubstanzen ausgeht bzw. abgegeben wird.

Anhand der Fig. 1 bis 4 werden konstruktive und funktionale Details der Vorrichtung 200 gemäß der Ausführungsform oder Varianten davon erläutert. Die Vorrichtung 200 hat mehrere Strahlungsquellen 210-1, 210-2, die jeweils zur Abgabe quasi-monochromatischer Anregungsstrahlung ausgebildet sind. Der Begriff "quasi-monochromatische Anregungsstrahlung" bezeichnet hierbei Anregungsstrahlung aus einem relativ engen Wellenlängenbereich, beispielsweise mit einer spektralen Bandbreite von deutlich weniger als 100 nm. Die spektrale Bandbreite kann zum Beispiel in der Größenordnung von ca. 10 nm oder wenigen 10 nm liegen. Die Anregung wird hier auch als schmalbandige Anregungsstrahlung bezeichnet. Wenn der Begriff "Anregungswellenlänge" gebraucht wird, so bezieht sich dieser dementsprechend auf einen relativ engen Wellenlängenbereich.

Im Beispielsfall weisen die Strahlungsquellen 210-1, 210-2 jeweils eine lichtemittierende Diode (LED) als aktives, Anregungsstrahlung emittierendes Element auf. Die Strahlungsquellen sind zur Steuerung und Leistungsversorgung an die Steuereinheit 115 angeschlossen. Die kleinflächigen, annähernd punktförmigen Strahlungsquellen sind räumlich getrennt voneinander an verschiedenen Orten in einer Strahlungsfläche 240 angeordnet. Die Strahlungsfläche ist diejenige Fläche, in der die Strahlungsquellen liegen. Die Strahlungsfläche kann, wie im Beispiel von Fig. 1, eine ebene Strahlungsfläche (Strahlungsebene) sein, so dass alle Strahlungsquellen in einer gemeinsamen Ebene liegen. Dies ist jedoch nicht zwingend. Die Strahlungsfläche kann auch einfach oder mehrfach gekrümmt sein. Die Strahlungsfläche kann so beschrieben werden, dass sie ein Muster von Strahlungsquellen in Abhängigkeit vom Ort darstellt, also eine gewisse örtliche Verteilung räumlich voneinander getrennter Strahlungsquellen. Zwischen den Strahlungsquellen existieren lateral Abstände ohne Strahlungsquelle.

Die Vorrichtung 200 umfasst einen Anregungsstrahlengang zur Abbildung von Anregungsstrahlung in die Indikatorfläche 130 in der Weise, dass Emissionen der Indikatorsubstanzen 125-1, 125-2 an denjenigen Orten oder Bereichen erzeugt bzw. angeregt werden können, an denen von den Strahlungsquellen emittierte Anregungsstrahlung auf die Indikatorfläche bzw. die dort angeordnete Indikatorsubstanz abgebildet wird.

Bei dem Ausführungsbeispiel weist der Anregungsstrahlengang mehrere Anregungskanäle auf, wobei jeder Anregungskanal ein erstes Abbildungssystem 220-1, 220-2 aufweist, das eine optische Achse 222-1, 222-2 des jeweiligen Anregungskanals definiert. Die Anregungskanäle sind in Fig. 1 lediglich mittels Linsensymbolen dargestellt, Einzelheiten und Besonderheiten werden anhand der nachfolgenden Figuren erläutert.

Der Begriff "Anregungskanal" bezeichnet hierbei die Gesamtheit aller optischen Komponenten, die dazu beitragen, Anregungsstrahlung von einer primären oder sekundären (virtuellen) Strahlungsquelle zur zugeordneten Indikatorsubstanz bzw. einem zugeordneten Ort oder Bereich der Indikatorfläche zu führen. Ein Abbildungssystem kann eine Einzellinse (zum Beispiel eine plankonvexe oder bikonvexe Linse, eine Zerstreuungslinse oder eine Balllinse) oder auch eine Linsengruppe mit zwei oder mehr Linsen enthalten. In einem Anregungskanal können auch ein oder mehrere Strahlformer und/oder ein oder mehrere wellenlängenselektive Elemente, wie zum Beispiel Filter, untergebracht sein.

Die von den Indikatorsubstanzen abgegebenen Emissionen werden über einen Emissionsstrahlengang geführt, der zur Abbildung der Indikatorfläche 130 in eine Empfangsfläche 245 der Vorrichtung 200 ausgelegt ist. Der Emissionsstrahlengang ist vorzugsweise so ausgelegt, dass im Wesentlichen nur die mit Anregungsstrahlung beaufschlagten Bereiche der Indikatorsubstanzen messtechnisch erfasst werden. Die Empfangsfläche kann eine ebene Fläche (Empfangsebene) sein, das ist jedoch nicht zwingend. Die Abbildung erfolgt derart, dass im Messbetrieb in der Empfangsfläche 245 ein Muster von Emissionen in Abhängigkeit vom Ort vorliegt. Dieses Muster korrespondiert im Beispielsfall hinsichtlich der räumlichen Verteilung von bestrahlten Bereichen mit dem Muster, welches die Emissionen an den durch Anregungsstrahlung beleuchteten Orten bzw. Bereichen in der Indikatorfläche 130 bilden. Die Muster sind einander somit im geometrischen Sinne ähnlich (was aber nicht zwingend ist). Die Abbildung der Anregungsstrahlung, die Anordnung der Indikatorsubstanzen und die Abbildung der Emissionen sind aufeinander abgestimmt.

Im Beispielsfall weist der Emissionsstrahlengang für alle Emissionen ein gemeinsames zweites Abbildungssystem 250 auf, das eine optische Achse 252 des Emissionsstrahlengangs definiert. Das zweite Abbildungssystem 250, das in Fig. 1 lediglich durch eine Linse symbolisiert ist, ist so konfiguriert, dass eintrittsseitig Emissionen von allen angeregten Indikatorsubstanzen empfangen und austrittsseitig auf bzw. in die Empfangsfläche 240 übertragen werden können. Der Emissionsstrahlengang kann als ein einziger Emissionskanal beschrieben werden, der bei einer einzigen gemeinsamen optischen Achse mehrere Sub-Kanäle für die von unterschiedlich angeordneten Indikatorsubstanzen kommenden Emissionen bereitstellt bzw. aufweist.

Der Empfangsfläche 245 ist eine optionale wellenlängenselektive Einrichtung 260 optisch nachgeschaltet. Die wellenlängenselektive Einrichtung hat eine Eintrittsfläche 262, die mit der in der Empfangsfläche empfangenen Emission beaufschlagbar ist. Die Eintrittsfläche kann mit der Empfangsfläche 245 zusammenfallen oder einen Abstand von dieser haben. Gegebenenfalls können ein oder mehrere optische Elemente zwischen der Empfangsfläche und Eintrittsfläche liegen. Es ist eine wesentliche Funktion der wellenlängenselektiven Einrichtung, aus dem in der Regel relativ breiten Spektrum der Emissionen, die auf die Empfangsfläche auftreffen, ein für die Erfassung und Auswertung besonders geeignetes oder gewünschtes engeres Spektrum zu selektieren bzw. auszuwählen. In der Austrittsfläche 264 der wellenlängenselektiven Einrichtung 260 liegen wellenlängenselektierte Emissionen vor.

In der Austrittsfläche 264 sind Empfänger 270-1, 270-2 zum Empfang von wellenlängenselektierten Emissionen und zur Wandlung dieser Emissionen in elektrische Signale angeordnet. Die Empfänger weisen entsprechende optoelektronische Komponenten auf, die signalleitend mit der in die Steuereinheit 115 integrierten Auswerteeinheit verbunden sind. Die von den Empfängern empfangenen elektrischen Signale werden in der Auswerteeinheit zur Charakterisierung der von den Empfängern empfangenen spektralen Information über die Emissionen ausgewertet.

Das Gerät 100 ist besonders für die Detektion von Analyten in Form von Explosivstoffen (Sprengstoffen) im Feldeinsatz ausgelegt. Dazu können insbesondere zeitliche Änderungen der Fluoreszenzintensität der Indikatorsubstanzen nach Bestrahlung mit Anregungsstrahlung erfasst und ausgewertet werden. Beispielsweise findet in vielen Fällen bei Kontakt eines Analyten mit einer darauf ansprechenden Indikatorsubstanz eine Fluoreszenzauslöschung (Quenching) statt, also eine Verminderung der von der Indikatorsubstanz abgegebenen Fluoreszenzintensität. In der Auswerteeinrichtung können dann z.B. Informationen über die Menge einer oder mehrerer gesuchter Zielsubstanzen (Analyten) und/oder Information über die Art der detektierten Zielsubstanzen ermittelt und beispielsweise über die Anzeigeeinrichtung 117 ausgegeben werden.

Eines der Ziele der vorliegenden Entwicklung ist es, spektroskopische Messungen von Emissionen mit hoher Empfindlichkeit in einem für den Feldeinsatz tauglichen, portablen Gerät zu realisieren. Ein wichtiger Beitrag dazu besteht darin, die für die Erzeugung und Führung von Anregungsstrahlung und Emissionsstrahlung erforderlichen optischen und elektrooptischen Komponenten so auszulegen und anzuordnen, dass die optische Vorrichtung 200 von der Bauform kompakt genug ist, um in ein handgehaltenes Gerät eingebaut werden zu können. Außerdem sollte die Vorrichtung 200 robust genug sein, um auch im rauen Feldeinsatz (beispielsweise bei oder nach Erschütterungen) dauerhaft funktionsfähig zu bleiben. Zudem werden eine hohe Messempfindlichkeit und hohe Messsicherheit angestrebt. Nachfolgend werden weitere Maßnahmen erläutert, die einzeln oder in Kombination miteinander für die Tauglichkeit des Geräts für den Feldeinsatz förderlich sind.

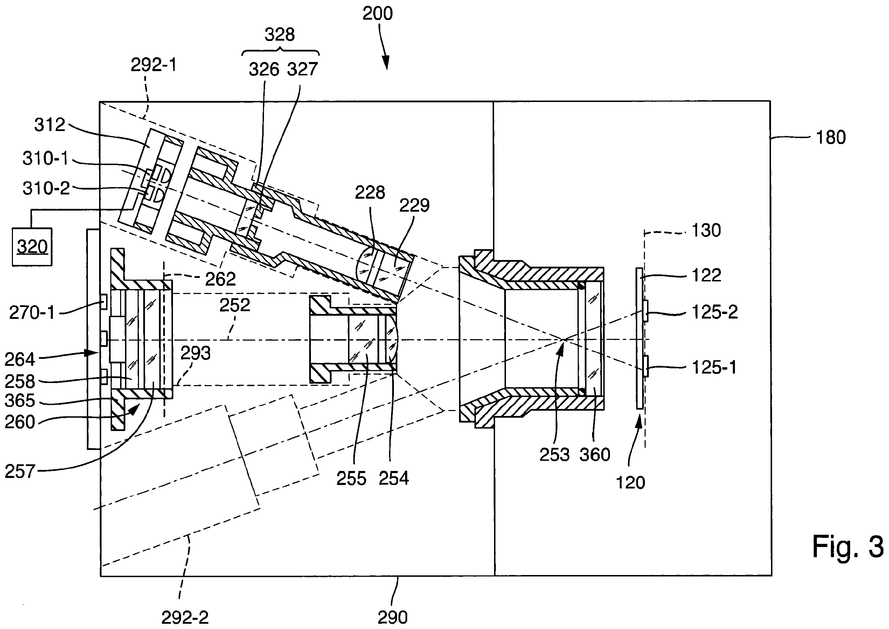

Fig. 2 zeigt in schrägperspektivischer Darstellung einiger Komponenten der Vorrichtung 200, die in richtiger räumlicher Anordnung zusammen mit der angekoppelten Haltevorrichtung 180 dargestellt sind (vgl. auch Fig. 3). Alle optischen Elemente der Vorrichtung 200, das heißt insbesondere alle zur Beeinflussung der Strahlung vorgesehenen optischen Elemente des Abbildungsstrahlengangs und alle zur Beeinflussung der Strahlung vorgesehenen optischen Elemente des Emissionsstrahlengangs, sind in einem einzigen massiven quaderförmigen Bauteil 290 integriert, das hier auch als Optikhalter 290 bezeichnet wird. Bei dem Bauteil handelt es sich im Beispielsfall um einen monolithischen Block aus einem geeigneten verwindungssteifen Material, beispielsweise einem Metall oder einer Keramik. Es kann sich um ein Spritzgussteil oder um ein Sinterteil handeln oder um ein Bauteil, welches durch materialabtragende Bearbeitung aus dem Vollen gearbeitet ist. Auch eine Herstellung im 3D-Druck ist möglich. Das Bauteil kann auch aus mehreren Teilen zusammengesetzt sein, die z.B. mittels Schrauben oder stoffschlüssig fest miteinander verbunden sind. Das Bauteil 290 bzw. die Vorrichtung 200 kann so klein sein, dass es bzw. sie in einen Würfel mit zehn Zentimetern Kantenlänge vollständig hineinpasst.

In dem Bauteil 290 sind mehrere rotationssymmetrische Aufnahmekanäle 292-1, 292-2, 293 zur Aufnahme der optischen Elemente der Anregungskanäle sowie des Emissionskanals ausgebildet. Die Längsmittelachse des Aufnahmekanals 293 für die optischen Elemente des Emissionskanals verläuft senkrecht zur ebenen Vorderseite 291 des Bauteils und (bei richtig angekoppelter Haltevorrichtung 180) senkrecht zur ebenen Indikatorfläche. Die Längsmittelachsen 294-1, 294-2, etc. der Aufnahmekanäle für die optischen Elemente der Anregungsstrahlengänge verlaufen schräg zu derjenigen des Emissionskanals und liegen auf einer Kegelmantelfläche, deren verjüngtes Ende der Haltevorrichtung 180 zugewandt ist. Der Kegelwinkel bzw. der Winkel zwischen den Längsmittelachsen von Emissionskanal und Anregungskanal liegt bei ca. 40°, kann aber auch kleiner (z.B. 20° oder mehr) oder größer (z.B. 60° oder weniger) sein. Die Längsmittelachsen schneiden sich mit Abstand vor der Indikatorfläche.

Die Vorrichtung 200 hat sechs gleichmäßig um den Emissionskanal herum verteilte separate, optisch voneinander getrennte Anregungskanäle und dementsprechend sechs gleichmäßig über die Kegelmantelfläche verteilte Aufnahmekanäle 292-1 etc.. Diese haben Abschnitte abgestufter, zum Indikator hin stufenweise kleiner werdender Durchmesser. Wie in Fig. 3 beispielhaft dargestellt, sind die optischen Elemente der Strahlengänge einzeln oder in Gruppen in hülsenförmigen Fassungselementen gefasst, die hier auch als Tubus (Einzahl) oder Tuben (Mehrzahl) bezeichnet werden können. Die Fassungselemente passen, vorzugsweise spielfrei, in die zugeordneten Abschnitte der Aufnahmekanäle im Bauteil 290 und werden beim Zusammenbau der Vorrichtung 200 nacheinander so in die Aufnahmekanäle geschoben und dort fixiert, dass die gehaltenen optischen Elemente mit hoher Präzision in ihren vorgesehenen Positionen entlang der Kanäle liegen.

Die optischen Achsen der ersten Abbildungssysteme für die Anregungsstrahlung fallen dann mit den Längsmittelachsen der Aufnahmekanäle 292-1 etc. des Optikhalters 290 zusammen, so dass auch die Anregungskanäle und der Emissionskanal eine kegelförmige Anordnung bilden, wobei die optischen Achsen der Anregungskanäle auf einer Kegelmantelfläche angeordnet sind, die den Emissionskanal umgibt. Die optische Achse des Emissionskanals ist dann in der Achse des Kegels angeordnet.

Die optischen Elemente sind durch Auswechslung der sie aufnehmenden Tuben leicht auswechselbar und können ggf. durch passende Tuben mit anderen optischen Elementen ersetzt werden, um das Gerät für eine andere Messaufgabe umzurüsten.

In Fig. 2 sind die räumlichen Anordnungen der Strahlungsquellen 210-1 etc. in der Strahlungsfläche 240, der Indikatorsubstanzen 125-1 etc. in der Indikatorfläche 120 und der Empfangsbereiche der Emissionsstrahlung in der Empfangsfläche 245 besonders anschaulich dargestellt. Die sechs separaten Strahlungsquellen 210-1 etc. bilden eine ringförmige Anordnung bzw. ringförmige Matrix in der ebenen Strahlungsfläche, die in der Nähe der Vorderseite 291 des Bauteils 290 liegt. Die Indikatorsubstanzen 125-1 etc. bilden ebenfalls eine ringförmige Anordnung, die in der ebenen Indikatorfläche 130 liegt. Nach Durchtritt der Emissionen durch den Emissionsstrahlengang bilden die Emissionen in der Empfangsfläche 245 ebenfalls ein ringförmiges Muster mit sechs räumlich voneinander separierten Bereichen, in denen Emissionsstrahlung auf die Empfängerfläche trifft. Das Muster der Emissionen in der Empfangsfläche 245 ist das integrale Ergebnis aus den Mustern der Strahlungsquellen, der Indikatorsubstanzen und der Emissionen. Dieses Muster ist durch das Muster der Strahlungsquellen und der den Strahlungsquellen nachgeordneten Optik bestimmt. Das heißt, dass das Muster der Emissionen in der Eintrittsfläche der wellenlängenselektiven Einrichtung vorgebbar ist durch die Anordnung der Strahlungsquellen, der optischen Elemente im Anregungsstrahlengang und die optischen Elemente im Emissionsstrahlengang.

Die Anordnung ist so getroffen, dass jede der Indikatorsubstanzen 125-1 etc. ein separater Empfänger 270-1 etc. zugeordnet ist. Im Beispielsfall sind also sechs separate Empfänger vorgesehen, so dass die Anzahl der Empfänger der Anzahl der effektiven Strahlungsquellen bzw. der Anzahl der Anregungskanäle entspricht. Zwischen den Empfängern liegen Zwischenräume ohne Empfänger. Die Empfänger sind separat justierbar. Als Empfänger sind im Beispielsfall halbleiterbasierte Photomultiplier, insbesondere auf Basis von Silizium (Si-Photomultiplier, Si-PMT) vorgesehen. Diese zeichnen sich durch hohe Empfindlichkeit und Robustheit gegen Erschütterungen aus. Alternativ können z.B. konventionelle Photomultiplier geringer Baugröße oder Photodioden verwendet werden. Bei der Anordnung gibt es keine zwei oder mehr Indikatorsubstanzen, denen derselbe Empfänger zugeordnet ist, so dass jeder Empfänger maximal Emissionen von genau einer Indikatorsubstanz empfangen kann. Hierdurch sind hochselektive Messungen möglich.

Der Anregungsstrahlengang und der Emissionsstrahlengang sind in Rückwärtsgeometrie angeordnet. Dies bedeutet, dass der Anregungsstrahlengang und der Emissionsstrahlengang auf derselben Seite des Indikators 120 bzw. der Indikatorfläche 130 angeordnet sind. Dies ermöglicht es, dass alle optischen Komponenten innerhalb der optischen Vorrichtung 200 bzw. innerhalb des Gehäuses 110 untergebracht werden können und dass der für die Messung vorgesehene Indikator 120 in einer vom Gehäuse abnehmbaren gesonderten Haltevorrichtung 180 untergebracht werden kann. Der im Betrieb ggf. gasführende Bereich der Halteeinrichtung 180 ist von dem Bereich der optischen Strahlengänge durch ein für die Anregungsstrahlung und die Emissionsstrahlung transparentes Schutzfenster 360 hermetisch getrennt (vgl. Fig. 3). Das leicht auswechselbare Schutzfenster wird von einer eigenen Fassungseinrichtung gehalten, die zwei ineinander steckbare hülsenförmige Fassungselemente umfasst.

Bei Messungen in Rückwärtsgeometrie kann grundsätzlich das Problem auftreten, dass spekular reflektierte Intensität der Anregungsstrahlung bis zum Empfänger gelangt und die Messempfindlichkeit bzw. das Signal/Rausch-Verhältnis verschlechtert. Spekular reflektierte Strahlung zeichnet sich dadurch aus, dass es sich um Strahlung handelt, deren Ausfallswinkel von einer reflektierenden Fläche (z.B. Grenzfläche Luft - Indikator) gleich deren Einfallswinkel ist. Bei Ausführungsformen der Erfindung sind besondere Vorkehrungen getroffen, um den Einfluss spekular reflektierter Strahlung auf die Messempfindlichkeit zu verringern.

Die Messgeometrie ist so eingestellt, dass vom Indikator spekular reflektierte und damit für die Emissionsmessung störende Strahlung praktisch nicht auf die Empfangsfläche treffen und damit die Messung stören kann. Dies wird zum einen dadurch erreicht, dass unterschiedliche Winkel der Anregung und der Emission bezüglich der Oberflächennormalen am Indikator auf der Auftrefffläche von Anregungsstrahlung vorgesehen sind. Während die Anregungsstrahlung schräg auf die ebene Indikatorfläche trifft, steht die optische Achse des Emissionsstrahlengangs senkrecht auf der Indikatorfläche. Zum anderen ist als Besonderheit in der Erfindung vorgesehen, dass sich die optischen Achsen der Abbildungssysteme des Anregungsstrahlengangs (erste Abbildungssysteme) und des Emissionsstrahlengangs (zweite Abbildungssysteme) nicht in der Indikatorfläche kreuzen, sondern mit deutlichem Abstand (z.B. in der Größenordnung von einem oder mehreren Millimetern) vor der Indikatorfläche, so dass Durchstoßpunkte der optischen Achsen in der Indikatorfläche auseinanderliegen bzw. räumlich voneinander getrennt sind.

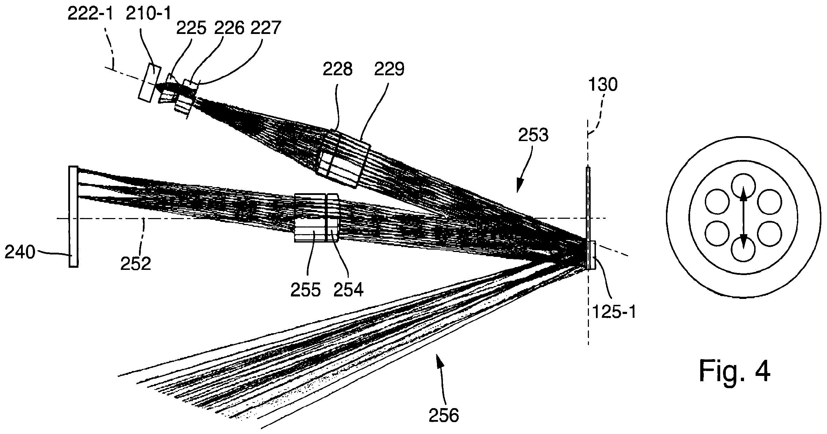

Zur Erläuterung zeigt Fig. 4 eine Darstellung in einer Ebene, die durch die optische Achse 252 des Emissionskanals und die optische Achse 222-1 eines einzigen Anregungskanals aufgespannt wird. Die von der Strahlungsquelle 210-1 ausgehende divergente Anregungsstrahlung wird zunächst durch eine Sammellinse 225 kollimiert und trifft danach auf einen Strahlformer 226 in Form eines Diffusors, der das zunächst ungleichmäßige Intensitätsprofil der Strahlung vergleichmäßigt. Dem Diffusor 226 ist eine Lochblende 227 nachgeschaltet, die durch die vom Diffusor austretende Anregungsstrahlung gleichmäßig ausgeleuchtet wird. Die Lochblende bildet somit eine (kreisrunde) sekundäre effektive Strahlungsquelle mit sehr gleichmäßigem Intensitätsprofil. Der Lochblende ist im geeigneten Abstand eine Einzellinse 228 in Form einer Plankonvexlinse nachgeschaltet, an deren Austrittsseite ein Anregungsfilter 229 folgt. Der Anregungsfilter ist als Bandpassfilter ausgelegt und hat die Aufgabe, aus dem schon relativ schmalbandigen Spektrum der von der primären Strahlungsquelle 210-1 abgegebenen Anregungsstrahlung einen für die Anregung der Indikatorsubstanz gewünschten engeren Wellenlängenbereich zu selektieren. Insbesondere werden längerwellige Strahlungsanteile (z.B. längerwellige Intensitätsausläufer von LEDs) geblockt, die in demjenigen Wellenlängenbereich liegen, in denen Fluoreszenz gemessen werden soll.

Diese schmalbandige Anregungsstrahlung trifft auf die erste Indikatorsubstanz 125-1 in der Indikatorfläche 130. Ein für die Auswertung vorgesehener Anteil der emittierten Strahlung (Emission) gelangt durch den Emissionskanal zu den Empfängern. Im Emissionskanal befindet sich das zweite Abbildungssystem, das hier ein einziges mit Brechkraft behaftetes optisches Element, nämlich eine Plankonvexlinse 254 aufweist, deren Austrittsseite ein Emissionsfilter 255 folgt. Der Emissionsfilter ist als relativ breitbandiges Bandpassfilter ausgelegt und hat u.a. die Aufgabe, Wellenlängen aus dem Wellenlängenbereich der Anregungsstrahlung (hier um ca. 370 nm) zu blocken. Die längerwellige Fluoreszenzstrahlung innerhalb der Emission wird dagegen im Wesentlichen unbeeinflusst transmittiert.

Es ist in Fig. 3 oder 4 gut ersichtlich, dass sich die optischen Achsen des Anregungsstrahlengangs und des Emissionsstrahlengangs an einem Kreuzungspunkt 253 mit Abstand vor der Indikatorfläche 130 kreuzen, so dass diejenige Anregungsstrahlung, die von der Strahlungsquelle 210-1 oberhalb des Emissionsstrahlengangs abgegeben wird, in der Indikatorfläche auf der gegenüberliegenden Seite des Emissionsstrahlengangs, nämlich unterhalb des Durchstoßpunkts der optischen Achse 252 des Emissionsstrahlengangs, auftrifft. Der Kreuzungspunkt kann z.B. einen oder mehrere Millimeter vor der Indikatorfläche liegen. Die Beaufschlagung des Indikators 120 in der Indikatorfläche 130 mit Anregungsstrahlung erfolgt also auf der optischen Achse des dem Anregungskanal zugehörigen Abbildungssystems (welches unter anderem die Linse 228 enthält). Die Anregung erfolgt unterhalb der optischen Achse des Abbildungssystems für die Emission. Das hat zur Folge, dass die von der Indikatorsubstanz 125-1 emittierte Emission, welche in den Emissionsstrahlengang fällt, durch das zweite Abbildungssystem (Abbildungssystem des Emissionsstrahlengangs) in einen räumlich begrenzten Bereich oberhalb der optischen Achse 252 des Emissionskanals abgebildet wird.

Dies ist zum einen vorteilhaft für den Betrieb der Vorrichtung 200 mit mehreren Anregungskanälen, die im Beispielsfall symmetrisch um die optische Achse des Emissionskanals herum angeordnet sind. Dadurch ergibt sich eine gute Ortsauflösung in der Empfangsfläche 245. Des Weiteren hat diese Anordnung den Vorteil, dass keinerlei vom Indikator spekular reflektierte Anregungsstrahlung in den Emissionskanal gelangen kann. Das bringt unter anderem den Vorteil, dass preiswerte Filter mit geringerer Blockung verwendet werden können oder dass auf Filter gänzlich verzichtet werden kann. Fig. 4 zeigt anschaulich, dass die spekular reflektierte Strahlung 256 nicht in die Apertur des Abbildungssystems des Emissionsstrahlengangs (zweites Abbildungssystem) fällt. Die Anregungsstrahlung läuft entlang der optischen Achse des Abbildungssystems des entsprechenden Anregungskanals. Die Emissionsstrahlung läuft dagegen nicht entlang der optischen Achse des Abbildungssystems des Emissionskanals, sondern kreuzt diese optische Achse zwischen der Indikatorfläche und der Empfangsfläche. Dies gilt für die Emissionsstrahlungen aller Kanäle. Diese verlaufen innerhalb des Abbildungssystems des Emissionsstrahlengangs in Sub-Kanälen, die jedenfalls im Bereich der Indikatorfläche 130 und der Empfangsfläche 245 räumlich voneinander separat liegen.

Es ist möglich, das Gerät 100 bzw. die optische Vorrichtung 200 mit nur einer einzigen Anregungswellenlänge zu betreiben. Die Anregungswellenlänge kann beispielsweise im nahen Ultraviolettbereich (zum Beispiel zwischen 350 nm und 400 nm) liegen, so dass die durch Rotverschiebung spektral zu längeren Wellenlängen verschobene Fluoreszenzstrahlung (Emissionsstrahlung) im sichtbaren Wellenlängenbereich liegen und durch entsprechende Empfänger detektiert werden kann. Es ist auch möglich, dass die Indikatorsubstanzen chemisch-physikalisch untereinander identisch sind, so dass mit der Vorrichtung zeitgleich sechs nominell identische Messungen durchgeführt werden könnten.

Es ist aber auch möglich, zwei oder mehr chemisch-physikalisch unterschiedliche Indikatorsubstanzen zu verwenden. Beispielsweise können unterschiedliche Indikatorsubstanzen für unterschiedliche Klassen oder Gruppen von Explosivstoffen optimiert sein. Die Vorrichtung kann beispielsweise so genutzt werden, dass drei unterschiedliche Arten von Indikatorsubstanzen in jeweils zwei der genutzten Positionen innerhalb der Indikatorfläche liegen. Diese können alle mit der gleichen Anregungswellenlänge bestrahlt werden. Es ist jedoch auch möglich, unterschiedliche, an die unterschiedlichen Indikatorsubstanzen angepasste Anregungswellenlängen zu nutzen.

Die Vorrichtung 200 kann so betrieben werden, dass alle Strahlungsquellen gleichzeitig aktiv geschaltet werden. Durch die Konfiguration ist sichergestellt, dass die Emissionen in der Eintrittsfläche der wellenlängenselektiven Einrichtung örtlich bzw. räumlich voneinander getrennt und damit so entkoppelt sind, dass die Emissionen ohne gegenseitige Überlagerung von Emissionen auflösbar sind. Durch die Konfiguration ist automatisch sichergestellt, dass die Beaufschlagung der Eintrittsfläche der wellenlängenselektiven Einrichtung mit mehreren Emissionen nicht zum selben Zeitpunkt am gleichen Ort erfolgt. Für die Empfänger ist eine örtliche Separierung vorgesehen, so dass es zu keiner Überlagerung verschiedener Spektren kommen kann.

Es ist bei der Anordnung auch möglich, in den unterschiedlichen Anregungskanälen wenigstens zum Teil mit unterschiedlichen Anregungswellenlängen zu arbeiten. So können beispielsweise zwei oder mehr Typen von Strahlungsquellen verwendet werden, die jeweils Anregungsstrahlung in unterschiedlichen Wellenlängenbereichen erzeugen. Beispielsweise können drei unterschiedliche Wellenlängenbereiche genutzt werden, wobei dann in jeweils zwei der Anregungskanäle die gleiche Anregungswellenlänge verwendet wird.

Anhand von Fig. 3 wird ein Ausführungsbeispiel erläutert, bei dem es möglich ist, einen einzigen Anregungskanal wahlweise mit zwei unterschiedlichen Anregungswellenlängen zu betreiben. An einem Träger 312 sind zwei Strahlungsquellen 310-1, 310-2 in Form von LEDs mit unterschiedlichen Anregungswellenlängen angebracht. Die Strahlungsquellen sind an einen Taktgenerator bzw. Taktgeber 320 angeschlossen, der ein funktionaler Bestandteil der Steuereinrichtung 115 des Geräts 100 sein kann. Mit dem Taktgenerator können die Lichtquellen separat voneinander geschaltet werden, insbesondere im Wechsel, so dass jeweils nur eine Anregungswellenlänge in den Anregungskanal eingekoppelt wird.

Die von den Strahlungsquellen abgehende divergente Strahlung trifft auf einen Strahlformer 326 in Form eines Diffusors. Diesem ist eine Lochblende 327 nachgeschaltet, deren Loch zentrisch zur optischen Achse sitzt. Die Anordnung ist so konfiguriert, dass unabhängig davon, welche der Strahlungsquellen 310-1, 310-2 eingeschaltet ist, das Loch der Lochblende gleichmäßig mit der Strahlung der eingeschalteten Strahlungsquelle ausgeleuchtet ist. Im Bereich des Lochs wird also eine sekundäre Strahlungsquelle mit derjenigen Anregungswellenlänge gebildet, die der Wellenlänge der eingeschalteten primären Strahlungsquelle entspricht.

Damit "sieht" das nachgeschaltete erste Abbildungssystem (hier mit einer einzigen Plankonvexlinse) nur einen einzigen Ort (Loch der Lochblende) für mehrere primäre Strahlungsquellen. Der Diffusor mit nachgeordneter Lochblende erfüllt somit die Funktion einer Strahlungsquellenvereinigungseinrichtung bzw. einer Strahlungsvereinigungseinrichtung 328. Die im Bereich der Lochblende gebildete virtuelle bzw. sekundäre Strahlungsquelle kann unterschiedliche Anregungswellenlängen abgeben, je nachdem, welche Strahlungsquelle durch den Taktgenerator aktiv geschaltet wird. Entsprechende Anordnungen mit drei oder mehr primären Strahlungsquellen sind ebenfalls möglich. Eine derartige Vervielfachung von effektiven Strahlungsquellen kann in einem einzigen Anregungskanal, in mehreren Anregungskanälen oder in allen Anregungskanälen vorgesehen sein.

Wie schon erwähnt, hat die wellenlängenselektive Einrichtung 260 unter anderem die Funktion, aus der auf die Empfangsfläche 245 auftreffenden Emissionsstrahlung einen engeren Wellenlängenbereich oder mehrere unterschiedliche engere Wellenlängenbereiche zu selektieren, deren Intensität dann mittels der Empfänger gemessen werden kann. Bei dem Beispiel von Fig. 3 weist die wellenlängenselektive Einrichtung 260 eine Bandpassfilteranordnung auf, die für alle Empfänger den gleichen Wellenlängenbereich hindurchlässt und nicht für die Auswertung gewünschte Strahlungsanteile blockt. Es ist auch möglich, eine wellenlängenselektive Einrichtung so auszulegen, dass für unterschiedliche Empfänger oder unterschiedliche Gruppen von Empfängern unterschiedliche Wellenlängenbereiche für den Empfang und die Auswertung durchgelassen werden. Somit können dann in einem breiteren Fluoreszenzspektrum mehrere relativ enge Wellenlängenbereiche bei Bedarf gleichzeitig gemessen und ausgewertet werden.

Im dargestellten Beispiel von Fig. 3 sind folgende Elemente im Emissionskanal angeordnet bzw. anordenbar. Eine Plankonvexlinse 254, ein optionaler Emissionsfilter 255 (gemeinsamer Filter für alle Empfänger), ein Kurzpassfilter 257 als gemeinsamer Filter für alle Empfänger, ein Langpassfilter 258 als gemeinsamer Filter für alle Empfänger. Bezugszeichen 365 bezeichnet eine Halterung für z.B. Bandpassfilter, die separat für jeden Empfänger vorgesehen sein können. Dieser Halter ist im Beispiel nicht belegt. Die Kombination von Kurzpassfilter 257 und Langpassfilter 258 kann bei Bedarf die Funktion eines Emissionsfilters übernehmen, so dass ein gesonderter Emissionsfilter entfallen kann.

Bei dem Ausführungsbeispiel können prinzipiell bis zu sechs unterschiedliche, spektral voneinander separierte Wellenlängenbereiche gemeinsam gemessen und ausgewertet werden, falls dies gewünscht ist. Wie in Fig. 3 zu erkennen, sind die Filterelemente der wellenlängenselektiven Einrichtung in einer eigenen Fassungseinrichtung 365 gefasst, die nach Abnehmen des Trägers für die Empfänger 270-1 etc. leicht auswechselbar ist. Somit können allein durch Auswechslung der wellenlängenselektiven Einrichtung unter Verwendung der gleichen Empfänger unterschiedliche Spektralbereiche für die Anwendung ausgewählt werden. Bewegliche Einrichtungen, wie z.B. ein Filterrad, sind nicht erforderlich.

Es gibt auch Ausführungsbeispiele, die ohne wellenlängenselektive Einrichtung auskommen, so dass die Emissionen in der Empfangsfläche ohne weitere spektrale Beschränkung in die Empfänger gelangen können. In diesem Fall können die aktiven Flächen der Empfänger unmittelbar in der Empfangsfläche 245 oder in deren Nähe angeordnet sein.

Wenn eine wellenlängenselektive Einrichtung vorgesehen ist, kann beispielsweise in deren Eintrittsebene der Spalt eines Gittermonochromators angeordnet sein. Es ist auch möglich, dass in der Eintrittsfläche eine Öffnung für einen definierten Durchgang der Emission in einen Filtermonochromator mit separaten Bandpassfiltern oder mit einem Verlaufsfilter lokalisiert ist. Zwischen der Empfangsfläche und der Eintrittsfläche können andere optische Elemente angeordnet sein. Beispielsweise kann dort ein Abbildungssystem mit einem Abbildungselement oder mehreren Abbildungselementen vorgesehen sein, um eine Abbildung der Emissionen der Empfangsfläche auf die Eintrittsfläche zu realisieren.

Das hier vorgeschlagene Konzept bietet die Möglichkeit, bei Bedarf bei einer größeren Anzahl von Anregungs- und Emissionswellenlängen zu messen. Damit kann sich ggf. der Informationsgehalt der Messwerte erhöhen. Da die Anzahl der Wellenlängen und die spektrale Auflösung im Vergleich zu komplett scannenden Spektrometern aufgrund deren Verwendung von schmalbandigen Lichtquellen begrenzt werden kann, können Vorrichtungen mit kleiner Baugröße bereitgestellt werden, die in einem feldtauglichen Gerät untergebracht werden können.

Bei manchen Ausführungsbeispielen werden drei bis neun Anregungswellenlängen im Ultrawellenspektralbereich (300 nm bis 400 nm) und drei bis neun Emissionswellenlängen im sichtbaren Spektralbereich (400 nm bis 700 nm) verwendet. Es wurde erkannt, dass ein vollständiger Wellenlängenscan für die im Vordergrund stehenden Anwendungen nicht erforderlich ist. Vielmehr reichen schmalbandige Wellenlängenbereiche, die aus einem breiteren Spektrum stammen können.

Der Indikator kann zwei oder mehr unterschiedliche Indikatorsubstanzen aufweisen, wodurch die Selektivität erhöht und Störungen reduziert werden können. Bei einer Verwendung mehrerer unterschiedlicher Indikatorsubstanzen (beispielsweise zwei, drei, vier oder fünf unterschiedliche Indikatorsubstanzen) können ggf. Änderungen der Emission besser charakterisiert und dadurch die Signalinterpretation genauer werden. Das kann zu einer Reduzierung von Ungenauigkeiten und zur Verbesserung der Messsicherheit auf dem Gebiet der Erkennung von Explosivstoffen und anderen gesundheits- und umweltrelevanten Substanzen führen.

Nachfolgend werden zahlreiche weitere Beispiele erläutert, die jeweils mit einer optionalen wellenlängenselektiven Einrichtung zwischen Empfangsfläche und den Empfängern (einer oder mehrere) ausgestattet sind. Der Anregungsstrahlengang der Beispiele hat jeweils nur einen einzigen Anregungskanal, der zeitgleich oder zeitlich gestaffelt von der Strahlung aller Strahlungsquellen (eine oder mehrere) genutzt wird. Die Ausführungsbeispiele nutzen vorzugsweise mehr als eine Anregungswellenlänge, z.B. zwei oder drei bis neun spektral unterschiedliche Anregungswellenlängen. Bei den schematischen Darstellungen werden für gleiche oder ähnliche Komponenten oder Merkmale jeweils die gleichen Bezugszeichen verwendet.

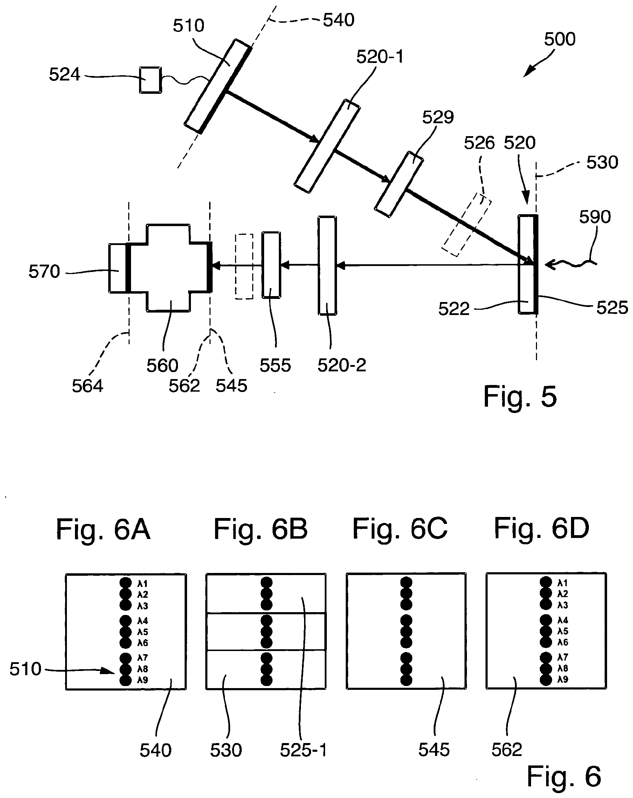

Fig. 5 zeigt eine schematische Darstellung eines Ausführungsbeispiels einer Vorrichtung 500 zur Erzeugung und Messung von Emissionen eines Indikators 520, der einen Träger 522 aufweist, auf dem mit Analyten 590 beaufschlagbare Indikatorsubstanzen 525 (ein oder mehrere) als dünne Schicht aufgebracht sind. Die Haltevorrichtung, die den Indikator trägt, ist nicht dargestellt. Die Indikatorsubstanzen befinden sich bei betriebsfertig montierter Vorrichtung bzw. betriebsfertig montiertem Gerät in der Indikatorfläche 530, die gekrümmt sein kann, im Beispielsfall aber eine Ebene (Indikatorebene) ist. In der (ebenen) Strahlungsfläche 540 befinden sich Strahlungsquellen 510 (eine oder mehrere). Diese sind jeweils schmalbandig und geben bei Aktivierung quasi-monochromatische Anregungsstrahlung ab. Ein optionaler Taktgeber 524 dient zum Ein- und Ausschalten einiger oder aller Strahlungsquellen, so dass in der Strahlungsfläche ein Muster von Strahlungsquellen in Abhängigkeit vom Ort und/oder in Abhängigkeit von der Zeit erzeugt werden kann.

Der Anregungsstrahlengang zur Abbildung von Anregungsstrahlung in die Indikatorfläche 530 hat im Beispielsfall nur einen einzigen Anregungskanal, der schräg zur Indikatorfläche verläuft und ein erstes Abbildungssystem 520-1 umfasst, das eine optische Achse des Anregungskanals definiert. Der Anregungskanal stellt mehrere Sub-Kanäle für zum Indikator laufende Anregungsstrahlung bereit. Das erste Abbildungssystem kann eine einzige Linse (Einzellinse) aufweisen, gegebenenfalls aber auch mehrere optische Elemente mit Brechkraft. Im Abbildungsstrahlengang befindet sich in der Regel noch ein Anregungsfilter 529, bei manchen Ausführungsformen auch noch ein optionaler erster Strahlformer 526.

Ein Emissionsstrahlengang dient zur Abbildung der Indikatorfläche 530 in eine Empfangsfläche 545, die im Beispielsfall eine ebene Fläche (Empfangsebene) ist. Der Emissionssstrahlengang zur Abbildung von Emissionsstrahlung hat nur einen einzigen Emissionskanal. Dieser stellt mehrere Sub-Kanäle für die vom Indikator zur Empfangsfläche laufenden Emissionen bereit. Der Emissionsstrahlengang umfasst ein zweites Abbildungssystem 520-2, dessen optische Achse im Beispielsfall senkrecht auf der ebenen Indikatorfläche 530 steht. Im Beispiel von Fig. 5 enthält der Emissionsstrahlengang noch einen Emissionsfilter 555, welcher bei anderen Varianten entfallen kann. Der Empfangsebene 545 folgt in Strahlungsrichtung eine wellenlängenselektive Einrichtung 560, deren Eintrittsfläche 562 in Fig. 5 mit der Empfangsfläche 545 zusammenfällt. In der Austrittsfläche 564 der wellenlängenselektiven Einrichtung oder in deren Nähe sind Empfänger 570 (einer oder mehrere) für die Emissionen angeordnet. Die Empfänger dienen dem Empfang von Emissionen aus der Empfangsfläche 545 und zur Wandlung der empfangenen Emissionen in elektrische Signale. Die Empfänger können beispielsweise als Photomultiplier, insbesondere als Silizium-Photomultiplier, ausgestaltet sein.

Wie bei den vorher beschriebenen Ausführungsformen können die optischen Elemente des Anregungsstrahlengangs und des Emissionsstrahlengangs in einem gemeinsamen Optikhalter integriert sein. Die Realisierung (mit Aufnahmekanälen für optische Elemente in einem einteiligen oder mehrteiligen Block) kann ähnlich sein wie bei den vorhergehenden Beispielen.

Die Vorrichtung 500 ist dafür ausgelegt, Emissionen bei einer größeren Anzahl von Anregungswellenlängen und Emissionswellenlängen zu erfassen. Damit kann bei bestimmten Anwendungen der Informationsgehalt gegenüber Varianten mit nur einer einzigen Anregungswellenlänge erhöht werden. Allerdings ist die Anzahl von nutzbaren Wellenlängen und die spektrale Auflösung im Vergleich zu scannenden Spektrometern bewusst durch die Vorrichtung begrenzt, so dass die Vorrichtung 500 in einem feldtauglichen Gerät zur Detektion von Explosivstoffen und/oder anderen umwelt- und gesundheitsgefährdenden Substanzen genutzt werden kann.

Bei den nachfolgend erläuterten Ausführungsbeispielen werden drei bis neun Anregungswellenlängen im ultravioletten Spektralbereich (300 nm bis 400 nm) und drei bis neun Emissionswellenlängen im sichtbaren Spektralbereich (400 nm bis 700 nm) verwendet. Ein vollständiger Wellenlängenscan ist nicht erforderlich und konstruktiv auch nicht vorgesehen. Ausreichende Messgenauigkeit wird bei der Vorrichtung durch die Verwendung schmalbandiger Wellenlängenbereiche aus einem breiteren Spektrum erzielt.

Der Indikator 520 kann mehrere Indikatorsubstanzen aufweisen und ist austauschbar, wodurch die Selektivität erhöht und Störungen reduziert werden können. Damit kann erreicht werden, dass die Änderungen der Emissionen besser charakterisiert und dadurch die Signalinterpretation genauer wird, was bei manchen Klassen von Analyten von Vorteil sein kann. Im Vergleich zu Lösungen des Standes der Technik kann dies zur Reduzierung von Ungenauigkeiten und zur Verbesserung der Messsicherheit auf dem Gebiet der Erkennung von Explosivstoffen und anderen gesundheits- und umweltrelevanten Substanzen genutzt werden.

Die Strahlungsquellen 510 in der Strahlungsfläche 540 sind für die entkoppelte Abgabe von Anregungsstrahlung zuständig, wobei sich diese Entkopplung sowohl auf den Ort als auch auf die Zeit beziehen kann. Die Anregungsstrahlung kann beispielsweise an einem Ort zu unterschiedlichen Zeiten oder an verschiedenen Orten zu einem Zeitpunkt oder auch an verschiedenen Orten zu verschiedenen Zeiten abgegeben werden. Die schmalbandige Anregungsstrahlung wird im Beispielsfall durch Spektralstrahler (LED) erzeugt, kann aber auch mithilfe wellenlängenselektiver Einrichtungen, wie zum Beispiel Gitter oder Filter, erzeugt werden.

Jeder mit einer Strahlungsquelle 510 belegte Ort in der Strahlungsfläche 540 repräsentiert eine bestimmte Anregungswellenlänge. Die Strahlungsfläche stellt somit ein Muster von Strahlungsquellen in Abhängigkeit vom Ort dar. Mithilfe des Taktgebers 524 können die Strahlungsquellen auch im Blitzbetrieb arbeiten, das heißt, sie sind zeitlich in definierter Weise mit dem Taktgenerator 524 taktbar (ein- und ausschaltbar). Damit stellt die Strahlungsfläche nicht nur ein Muster von Strahlungsquellen 510 in Abhängigkeit vom Ort dar, sondern auch ein Muster von Strahlungsquellen 510 in Abhängigkeit von der Zeit. Die Strahlungsfläche kann eben oder gekrümmt ausgebildet sein. Im Beispielsfall handelt es sich um eine Strahlungsebene.

In Fig. 6 sind für ein Ausführungsbeispiel die Verhältnisse in der Strahlungsfläche 540, der Indikatorfläche 530, der Empfangsfläche 545 und der Eintrittsfläche 562 der wellenlängenselektiven Einrichtung 560 dargestellt.

Im Beispielsfall besteht der Indikator im Wesentlichen aus einem Träger, auf dem mehrere Indikatorsubstanzen 525-1 bis 525-3 in Form nebeneinanderliegender Streifen aufgebracht sind. Die Indikatorfläche stellt somit ein Muster von (drei) Indikatorsubstanzen in Abhängigkeit vom Ort dar. Die Strahlungsquellen sind durch geeignete Maßnahmen, nämlich durch den Aufbau des Abbildungsstrahlengangs, in die Indikatorfläche abbildbar. Die Indikatorsubstanzen werden auf diese Weise durch die Anregungsstrahlung zur Emission angeregt. Die Emission der Indikatorsubstanzen wird genau an denjenigen Orten bzw. kleinflächigen Bereichen erzeugt, wo die Anregungsstrahlung auf die Indikatorfläche abgebildet wird. Die Indikatorfläche stellt damit nicht nur ein Muster von Indikatorsubstanzen in Abhängigkeit vom Ort dar, sondern auch ein Muster von Emissionen in Abhängigkeit von der Zeit. Dabei sind beide Muster aufeinander abstimmbar in der Weise, dass zum einen die Indikatorsubstanzen 525-1 etc. jeweils von Strahlungsquellen verschiedener Wellenlängen zur Emission angeregt werden. Zum anderen ist die Orts- und Zeitabhängigkeit der Emissionen so eingestellt, dass damit die Eigenschaften der wellenlängenselektiven Einrichtung (beispielsweise die Art der Eintrittsfläche und der Austrittsfläche der wellenlängenselektiven Einrichtung 560 sowie die Art der Empfänger 570) berücksichtigt werden können. Die Konfiguration ist derart, dass die spektral aufgelösten Emissionen ohne gegenseitige Überlagerung an den Empfängern messbar sind.

Die Muster der Strahlungsquellen und der Indikatorsubstanzen sind an die Strömungsverhältnisse bzw. an die Geometrie der Beaufschlagung des Indikators mit dem Analyten 590 anpassbar und umgekehrt. Die Beaufschlagung kann, wie bereits dargestellt, zum Beispiel in Form eines geführten Luftstroms erfolgen. Der Indikator 520 wird, wie bei den anderen Ausführungsbeispielen beschrieben, mithilfe der Haltevorrichtung mit der Vorrichtung 500 in der Weise mechanisch gekoppelt, dass die Indikatorsubstanzen 525-1 etc. exakt und definiert in der Indikatorfläche 530 positioniert sind. Der Indikator ist über diese Haltevorrichtung leicht auswechselbar.

Die von den Indikatorsubstanzen ausgehenden Emissionen werden durch geeignete Maßnahmen, nämlich den Aufbau des Emissionsstrahlengangs, in die Empfangsfläche definiert abgebildet. Die Empfangsfläche 545 stellt somit ein Muster von Emissionen in Abhängigkeit von Ort und von der Zeit dar. Die Emissionen können direkt von der Empfangsebene zur Eintrittsebene der wellenlängenselektiven Einrichtung gelangen oder durch zwischengeschaltete optische Mittel beeinflusst werden. Mithilfe der wellenlängenselektiven Einrichtung 560 erfolgt eine spektrale Auflösung aller Emissionen, die dann auf definierte Orte der Austrittsfläche der wellenlängenselektiven Einrichtung gerichtet werden. Dort befinden sich die Empfänger 570 (ein oder mehrere). Durch die Beaufschlagung der Austrittsfläche mit spektral aufgelösten Emissionen an definierten Orten und/oder zu definierten Zeiten (durch die zeitliche Taktung der Strahlungsquellen) kann erreicht werden, dass die spektral selektierten Emissionen von jedem Ort der Indikatorfläche separat und ohne gegenseitige Überlagerung von den Empfängern (einer oder mehrere) empfangen und in Signale umgewandelt werden können.

Die Taktung mithilfe des Taktgebers 524 kann unterschiedlich durchgeführt werden. Beispielsweise können alle Strahlungsquellen zeitlich versetzt nacheinander (das heißt seriell) oder ein Teil davon auch gleichzeitig getaktet werden. Die Konfiguration ist so eingerichtet, dass stets die Bedingung eingehalten wird, dass die Beaufschlagung des oder der Empfänger 570 mit mehreren Emissionen nicht zum selben Zeitpunkt am gleichen Ort erfolgen kann. Für die Empfänger kann eine örtliche und/oder eine zeitliche Separierung vorgegeben werden, damit es zu keiner Überlagerung unterschiedlicher Spektren kommt. Beispielsweise können mehrere Empfänger in einem eindimensionalen oder zweidimensionalen Empfänger-Array gemäß einem bestimmten Muster mit Abstand zueinander in der Austrittsfläche angeordnet sein.

Die gesamte Vorrichtung 500 ist modular aufgebaut. So können beispielsweise Strahlungsquellen 510, Indikatoren 520 und/oder Empfänger 570 ohne Eingriff in den Anregungsstrahlengang und/oder den Emissionsstrahlengang ausgetauscht werden. Die spektralen Bandbreiten zum Beispiel des Anregungsfilters und/oder des Emissionsfilters und/oder der wellenlängenselektiven Einrichtung können durch Austausch dieser Komponenten geändert werden. Die Vorrichtung 500 ist auch dafür geeignet, nur mit einem einzigen Indikator mit einer einzigen Indikatorsubstanz und mit nur einer einzigen Strahlungsquelle ausgestattet zu werden. Die Vorrichtung 500 kommt ohne bewegliche Teile aus. Dies trägt zur Robustheit der Vorrichtung und für die Tauglichkeit für den Feldeinsatz bei. Weiterhin ist kein geometrischer oder physikalischer Strahlteiler erforderlich oder vorgesehen. Dies ist u.a. förderlich für die Messempfindlichkeit.

Bei der Vorrichtung 500 ist das Muster der Emissionen in der Eintrittsfläche 562 der wellenlängenselektiven Einrichtung durch Art und gegebenenfalls Verteilung von Strahlungsquellen sowie die abbildenden optischen Systeme im Anregungsstrahlengang und Emissionsstrahlengang vorgebbar. Im Beispielsfall ist die Strahlungsfläche 540 außerhalb der einfachen Brennweite des ersten Abbildungssystems 520-1 und die Indikatorfläche 530 außerhalb der einfachen Brennweite des zweiten Abbildungssystems 520-2 lokalisiert. Damit kann erreicht werden, dass das erste Abbildungssystem die Strahlungsfläche in die Indikatorfläche abbildet. Das Muster der Strahlungsquellen 510 findet sich dann auf der Indikatorfläche wieder und erzeugt ein entsprechendes Muster von Emissionen. Das zweite Abbildungssystem 520-2 bildet die Indikatorfläche in die Empfangsfläche rückwärtig ab. Das Muster der Emissionen findet sich dann in der Empfangsebene 545 wieder. Die Ebenen liegen jeweils mit Abstand auseinander.

Der Indikator 520 kann aufgrund dieser Anordnung in Rückwärtsgeometrie besonders einfach als separates Element konstruiert und hermetisch trennbar von der optischen Vorrichtung 500 ausgebildet sein. Das kann unter anderem dann von Vorteil sein, wenn der Indikator ausgewechselt werden muss, ohne dabei optische Elemente zu beschädigen oder zu verschmutzen. Außerdem ist es dadurch auch möglich, den Indikator zwecks Adsorption und Desorption von Analyten einer definierten Wärmebehandlung auszusetzen, ohne den Messvorgang zu stören.

In der Vorrichtung 500 ist die Strahlungsfläche 540 entlang und/oder quer zur optischen Achse des ersten Abbildungssystems 520-1 justierbar. Die Indikatorfläche 530 ist entlang und/oder quer zu den optischen Achsen der ersten und zweiten Abbildungssysteme justierbar. Eine Justierbarkeit entlang und/oder quer zu einer optischen Achse bedeutet dabei, dass der Abstand zwischen der Strahlungsfläche und dem Abbildungssystem sowie der Abstand und der Winkel zwischen Strahlungsfläche und der optischen Achse des ersten Abbildungselements eingestellt werden kann. Dies gilt analog für die Indikatorfläche. Die Messgeometrie kann damit so eingestellt werden, dass vom Indikator 520 spekular reflektierte und damit für die Emissionsmessung störende Strahlung nicht in die Empfangsfläche treffen und damit auch nicht in die wellenlängenselektive Einrichtung 560 gelangen kann (vgl. Fig. 4).

Außerdem kann erreicht werden, dass unterschiedliche Muster der Emission auf der Indikatorfläche realisiert werden können. So kann beispielsweise die Größe (Durchmesser) der einzelnen Elemente des Musters durch definierte Abstände der Strahlungsfläche zum ersten Abbildungssystem 520-1 eingestellt bzw. verstellt werden. Es kann dann von Vorteil sein, wenn die Größe der einzelnen Elemente der Emission (Spots entsprechend der Abbildung der einzelnen Strahlungsquellen in die Indikatorfläche) auf die flächenhafte Ausdehnung der einzelnen auf den Träger aufgebrachten Indikatorsubstanzen angepasst werden soll.

Der Anregungsfilter 529 und der Emissionsfilter 555 können die gleichen Funktionen haben wie bereits im Zusammenhang mit dem ersten Ausführungsbeispiel beschrieben. Unter Verwendung dieser Komponenten können Emissionsmessungen mit großem Signal-Rausch-Verhältnis durchgeführt werden.

Bei der wellenlängenselektiven Einrichtung 560 kann es sich um einen Filtermonochromator mit einem oder mehreren Kantenfiltern handeln. Es wäre auch möglich, einen Gittermonochromator als wellenlängenselektive Einrichtung zu nutzen. Bei einem Gittermonochromator ist die Eintrittsebene häufig in Form eines Spalts ausgebildet, über den die Emissionen einkoppeln müssen. Im Beispielsfall von Fig. 6 sind die Strahlungsquellen 510 gemäß Fig. 6A in Form einer geraden Reihe bzw. einer Spalte angeordnet. Über das erste Abbildungssystem 520-1 werden diese Strahlungsquellen in die Indikatorfläche 530 gemäß Fig. 6B abgebildet. In der Indikatorfläche liegen im Beispielsfall drei Indikatorsubstanzen, die in Form nebeneinanderliegender Streifen auf dem Träger horizontal aufgetragen sind. Jede Indikatorsubstanz wird mit Anregungsstrahlung bei drei unterschiedlichen Wellenlängen λi beaufschlagt. Es entsteht ein Muster von Anregungsstrahlung, das als Spalte (eindimensionale Matrix) ausgebildet ist. Durch die Abbildung über den Emissionsstrahlengang entsteht ein entsprechendes Muster (eindimensionale Matrix) von Emissionen in der Empfangsebene 545 (Fig. 6C). Dies sind die Bilder der in der Indikatorfläche beleuchteten Bereiche der Indikatorsubstanzen. Die Anordnung ist so justiert, dass alle Emissionen dieser Reihe in den Eintrittsspalt des Gittermonochromators fallen.

Die Abbildungen der Emissionen in die Eintrittsfläche sind gemäß Fig. 6C oder 6D in drei Gruppen λ1 bis λ3, λ4 bis λ6 und λ7 bis λ9 angeordnet. Jede Gruppe repräsentiert die Emissionen einer Indikatorsubstanz bei drei Wellenlängen der Anregungsstrahlung. Die Größe der ausgeleuchteten "Spots" in der Indikatorfläche kann beispielsweise im Bereich von ein bis zwei Millimetern liegen, davon aber auch substanziell abweichen. Die zeitliche Taktung der Strahlungsquellen mit dem Taktgenerator ist so konfiguriert, dass in jeder Gruppe die Strahlungsquellen zeitlich nacheinander angesteuert werden. Somit gibt es keine Überlagerung verschiedener spektral aufgelöster Emissionen auf einen zugeordneten Empfänger. Durch die Konfiguration kann erreicht werden, dass jede Indikatorsubstanz bei drei unterschiedlichen Anregungswellenlängen und je nach Art des Monochromators bei mindestens drei Emissionswellenlängen gemessen werden kann. Damit ergibt sich ein hoher Informationsgehalt für die Auswertung.

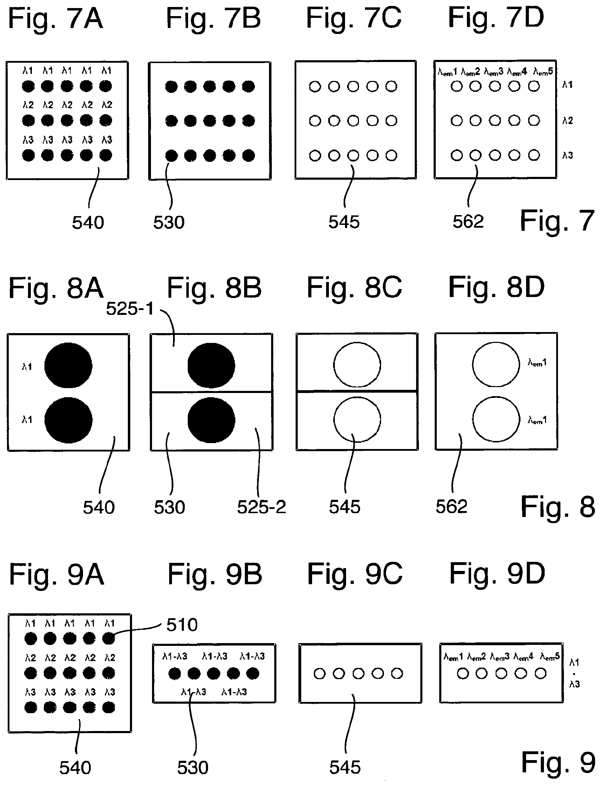

Es sind auch andere räumliche und/oder zeitliche Muster der Anordnung von Strahlungsquellen, Emissionen und/oder Indikatorsubstanzen und Kombinationen davon möglich. Bei dem schematischen Beispiel von Fig. 7 wird die zugehörige Vorrichtung so aufgebaut, dass als wellenlängenselektives Element ein Filtermonochromator mit einem oder mehreren spektralselektiven Filtern genutzt wird. Die Strahlungsquellen sind gemäß Fig. 7A in Form einer rechteckigen Matrix mit insgesamt 15 separat nutzbaren Strahlungsquellen angeordnet. Dabei weisen die Strahlungsquellen in derselben Zeile jeweils die gleichen Anregungswellenlängen (λ1, λ2 oder λ3) auf, die Strahlungsquellen in derselben Spalte, jedoch unterschiedliche Anregungswellenlängen. Der Einfachheit halber werden hier drei verschiedene Anregungswellenlängen λ1 bis λ3 verwendet. Die Abbildung in die Indikatorfläche führt dazu, dass die ausgeleuchteten Spots ebenfalls als zweidimensionale 3x5-Matrix vorliegen (Fig. 7B). Der Indikator trägt hier nur eine einzige Indikatorsubstanz, die an insgesamt 15 räumlich voneinander entfernt liegenden Orten bzw. Bereichen mit insgesamt drei unterschiedlichen Anregungswellenlängen bestrahlt wird. Die Fläche der aufgetragenen Indikatorsubstanz kann jedoch auch nur im Wesentlichen der jeweils bestrahlten Fläche entsprechen. Durch die Abbildung im Emissionsstrahlengang liegt auch die Empfangsebene 545 mit einem Muster abgebildeter Emissionen in Form einer 3x5-Matrix vor. Die wellenlängenselektive Einrichtung hat ein Array von Filtern, wobei das Muster der Filter mit dem Muster der abgebildeten Emissionen übereinstimmt. Die Filter können beispielsweise in derselben Zeile unterschiedliche Wellenlängen und die Filter in derselben Spalte gleichen Wellenlängen transmittieren. Die Wellenlängen der Filter sind auf die Wellenlängen der Emissionen abgestimmt. Da üblicherweise der Wellenlängenbereich der Emissionen bei längeren Wellenlängen liegt als der Wellenlängenbereich der Anregungsstrahlung, sind die Wellenlängen der Filter λₑₘ₁ bis λₑₘ₅ größer als die Wellenlängen λ1 bis λ3 der Anregungsstrahlung.

So können beispielsweise die Anregungswellenlängen λ1 bis λ3 im Bereich 350 nm bis 400 nm und die Emissionswellenlängen λₑₘ₁ bis λₑₘ₅ im Bereich 450 nm bis 650 nm angeordnet sein.

In der Austrittsfläche der wellenlängenselektiven Einrichtung ist wieder ein Empfängerarray mit insgesamt 15 Empfängern angeordnet. Diese sind so örtlich verteilt, dass das Muster der Empfängerelemente mit dem Muster der abgebildeten Emissionen bzw. mit dem Muster der Filter übereinstimmt. Somit ist jedem Empfängerelement genau ein Filter zugeordnet. Der Empfänger besteht im Beispielsfall also aus drei übereinanderliegenden Zeilen, die jeweils fünf Empfängerelemente enthalten (3x5-Empfängerarray). Die spektrale Auflösung der Emissionen in dieser Anordnung erfolgt in Zeilenrichtung. Jede Zeile repräsentiert eine definierte Anregungswellenlänge, so dass sich spektral aufgelöste Emissionen bei fünf verschiedenen Emissionswellenlängen und drei verschiedenen Anregungswellenlängen ergeben. Bei dieser Anordnung ist eine zeitliche Entkopplung der Strahlungsquellen nicht erforderlich, alle Strahlungsquellen können mit dem Taktgenerator gleichzeitig angesteuert werden.

Wenn anstelle eines Empfängerarrays mit mehreren separaten Empfängern nur ein einziger Empfänger verwendet werden soll, können die Strahlungsquellen nacheinander getaktet werden. Der Indikator kann auch mehrere Indikatorsubstanzen aufweisen.

Anhand Fig. 8 wird ein Ausführungsbeispiel erläutert, bei dem in der Indikatorfläche 530 genau zwei verschiedene Indikatorsubstanzen 525-1 und 525-2 vorgesehen sind und die wellenlängenselektive Einrichtung mit einem oder mehreren spektralselektiven Filtern aufgebaut ist. Es werden zwei Strahlungsquellen 510 genutzt, die gemäß Fig. 8A mit Abstand zueinander in einer Spalte angeordnet sind. Eine entsprechende Anordnung ergibt sich in der Indikatorfläche (Fig. 8B), in der genau zwei verschiedene Indikatorsubstanzen angebracht sind. Durch die Abbildung im Emissionsstrahlengang entsteht in der Empfangsfläche bzw. der Eintrittsfläche der wellenlängenselektiven Einrichtung das in Fig. 8C gezeigte Muster von zwei mit Abstand zueinender liegenden Emissionen. Die Filterung erfolgt für beide Emissionen im gleichen Wellenlängenbereich, wozu ein gemeinsamer Filter oder ein Filterarray genutzt werden kann. Das zugeordnete Empfängerarray besteht aus zwei separaten Empfängerelementen. Die Strahlungsquellen können mit dem Taktgenerator gleichzeitig angesteuert werden. Es ist auch eine Taktung möglich. So kann prinzipiell ein einziger Empfänger für beide Emissionen verwendet werden, wenn dann die Strahlungsquellen nacheinander getaktet werden, so dass eine zeitliche Entkopplung realisiert wird.

Es ist prinzipiell möglich, im Anregungsstrahlengang und/oder im Emissionsstrahlengang zusätzlich zu einem Abbildungssystem auch noch eine nicht abbildende Optik bzw. einen Strahlformer zu nutzen, der ebenfalls justierbar sein kann. Im Beispielsfall von Fig. 9 wird in der zugehörigen Vorrichtung 500 ein Strahlformer genutzt, um bei Verwendung einer zweidimensionalen 3x5-Matrix von 15 Strahlungsquellen 510 in der Strahlungsfläche 540 im Bereich der Empfangsfläche 545 eine Anordnung der Emissionen in nur einer einzigen Reihe (eindimensionale Matrix) zu erzielen. Es wird also mittels eines Strahlformers eine Bildwandlung integriert. Im Beispielsfall ist im Anregungsstrahlengang zwischen dem ersten Abbildungssystem 520-1 und der Indikatorfläche 530 ein Strahlformer 526 in Form einer Zylinderlinse eingefügt. Dadurch kann eine zweidimensionale Matrixanordnung von Strahlungsquellen 510 in eine eindimensionale Matrix, das heißt eine Reihe von beleuchteten Spots, abgebildet werden. Die Empfänger eines Empfängerarrays können dementsprechend dann auch in einer Reihe angeordnet sein.

Die Strahlungsquellen können für die verschiedenen Anregungswellenlängen λ1 bis λ3 mit dem Taktgenerator nacheinander angesteuert werden. Strahlungsquellen gleicher Anregungswellenlänge (also in einer Reihe nebeneinanderliegende Strahlungsquellen) können gleichzeitig getaktet werden.

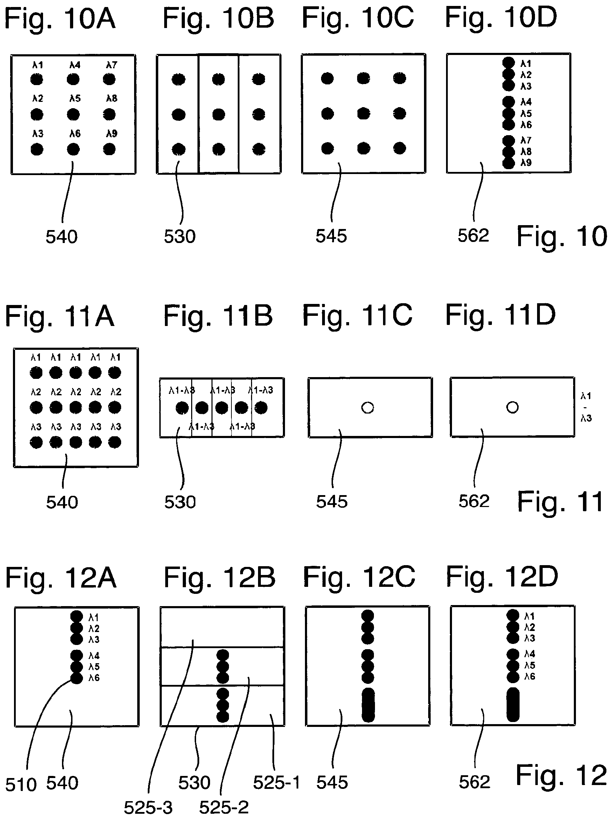

Sofern ein Strahlformer zur Umverteilung der Anordnung von Anregungsstrahlung und/oder Emissionen gewünscht ist, kann auch ein Lichtwellenleiterbündel als Strahlformer genutzt werden. Im Beispiel von Fig. 10 findet eine solche Strahlformung zwischen der Empfangsfläche 545 und der im Strahlengang folgenden Eintrittsfläche 562 einer wellenlängenselektiven Einrichtung statt, die mit optischem und räumlichem Abstand hinter der Empfangsfläche liegt. In der Strahlungsfläche 540 werden insgesamt neun Strahlungsquellen mit insgesamt neun Anregungswellenlängen genutzt. Die Strahlungsquellen sind in Form einer zweidimensionalen 3x3-Matrix angeordnet. Die Indikatorsubstanzen liegen in Form dreier separater Streifen unterschiedlicher Indikatorsubstanzen vor. Diese werden zeitlich aufeinanderfolgend mit jeweils drei der Wellenlängen angeregt. In der Empfangsfläche 545 ergibt sich somit ein Muster von Emissionen in Abhängigkeit vom Ort, ebenfalls in Form einer 3x3-Matrix. Die Strahlformung zwischen der Empfangsfläche 545 und der Eintrittsfläche 562 der wellenlängenselektiven Einrichtung 560 ist dann so gewählt, dass die neun Emissionen in drei Gruppen mit jeweils drei unterschiedlichen Wellenlängen in einer einzigen Reihe bzw. Spalte angeordnet sind (Fig. 10D). In dieser Konfiguration kann ein Gittermonochromator oder ein Filtermonochromator als wellenlängenselektive Einrichtung genutzt werden.

Anhand von Fig. 11 wird eine Variante erläutert, bei der sowohl im Anregungsstrahlengang (zwischen Strahlungsfläche 540 und Indikatorfläche 530) als auch im Emissionsstrahlengang (zwischen Indikatorfläche 530 und Empfangsebene 545) ein Strahlformer bzw. eine nichtabbildende Optik angeordnet ist. Beispielsweise kann eine Zylinderlinse als Strahlformer im Anregungsstrahlengang und im Emissionsstrahlengang genutzt werden. Die Strahlungsquellen (Fig. 11A) sind wieder in Form einer 5x3-Matrix angeordnet, wobei drei Anregungswellenlängen genutzt werden. Durch den Strahlformer im Anregungsstrahlengang erfolgt eine Abbildung auf die Indikatorfläche 530, in der sich fünf unterschiedliche Indikatorsubstanzen in Form von nebeneinanderliegenden Streifen befinden. Die Abbildung erfolgt so, dass jede der Indikatorsubstanzen zeitlich separiert mit jeder der drei Anregungswellenlängen bestrahlt werden kann. Die Strahlungsquellen werden in Form einer einzigen Reihe auf die Indikatorsubstanzen abgebildet. Der Strahlformer in Form einer Zylinderlinse innerhalb des Emissionsstrahlengangs ist so gestaltet, dass die Emissionen der Indikatorfläche in der Empfangsfläche 545 auf einen einzigen Punkt (Spot mit endlicher Fläche) abgebildet werden. Das zweite Abbildungssystem besteht dabei aus mehreren Linsen, nämlich einem Linsenarray, welches als Teiler ausgebildet ist, wobei jeder der Indikatorsubstanzen in der Indikatorfläche und damit jeder Emission eine Linse zugeordnet ist, die die divergente Strahlung der Emission kollimiert. Der nachgeschalteten Zylinderlinse wird somit Parallelstrahlung an fünf verschiedenen Orten als Zeile zugeführt. Die Zylinderlinse ist so ausgerichtet, dass sie die Richtung der Emissionen links und rechts der mittleren Emission ändert, nicht jedoch die Richtung der mittleren Emission. Die Zylinderlinsen von Anregungsstrahlengang und Emissionsstrahlengang sind um 90° gegeneinander verdreht.

Als wellenlängenselektive Einrichtung kann z.B. ein Gittermonochromator genutzt werden, dessen Eintrittsspalt punktförmig bzw. kreisförmig mit definierter Fläche ausgebildet ist. Über diese Öffnung koppelt die Emission in der Eintrittsfläche 562 immer am selben Ort in die wellenlängenselektive Einrichtung ein. Im Gittermonochromator erfolgt eine spektrale Auflösung der Emission, die zeilenförmig ausgebildet ist. In der Austrittsfläche des Gittermonochromators ist ein zeilenförmiges Empfängerarray angeordnet, dessen Empfänger an die spektrale Auflösung der Emission angepasst sind.

Die Strahlungsquellen werden in diesem Fall für die verschiedenen Anregungswellenlängen λ1 bis λ3 mit dem Taktgenerator in der Weise nacheinander angesteuert, dass jeweils nur eine einzige Indikatorsubstanz in der Indikatorfläche mit Anregungsstrahlung bei nur einer einzigen Wellenlänge beaufschlagt wird. Damit kann erreicht werden, dass die spektral aufgelösten Emissionen separat, ohne sich störend zu überlagern, in die wellenlängenselektive Einrichtung gelangen und mit den Empfängern gemessen werden können.

Anhand von Fig. 12 wird eine weitere Variante erläutert. Die Emission der Indikatoren kann einerseits durch Anregungsstrahlung erzeugt werden (Fluoreszenz, Phosphoreszenz). Andererseits kann die Emission auch durch chemische Reaktionen verursacht sein (Chemilumineszenz). Deshalb ist bei dieser Variante in der Indikatorfläche 530 neben zwei fluoreszenzfähigen Indikatorsubstanzen 525-1, 525-2 auch (mindestens) eine chemilumineszenzfähige Indikatorsubstanz 525-3 angeordnet. Das Muster der Emissionen in der Eintrittsfläche der wellenlängenselektiven Einrichtung ist hier durch die Strahlungsquellen 510, abbildende Optik und durch die Anordnung der chemilumineszierenden Indikatorsubstanzen in der Indikatorfläche vorgebbar. Die Emission der chemilumineszierenden Substanzen wird durch eine chemische Reaktion zwischen den Analyten und den Indikatorsubstanzen erzeugt. Für diese Form der Lumineszenz ist somit eine Anregungsstrahlung nicht erforderlich. Es können eine oder mehrere Indikatorsubstanzen in definierter Weise auf dem Träger aufgebracht sein. Dabei liegt dieser Bereich außerhalb des Bereiches, der mit Anregungsstrahlung der Strahlungsquellen für die Emission in Form der Fluoreszenz beaufschlagbar ist. Damit können die durch Analyten erzeugten Emissionen an Orten in der Empfangsebene abgebildet werden, die von mit Anregungsstrahlung erzeugten Emissionen frei sind. Es finden keine störenden Überlagerungen der verschiedenen Emissionen statt. Somit sind unterschiedliche Indikatoren, deren Emissionen einerseits durch Anregungsstrahlung und andererseits durch Analyten durch eine chemische Reaktion erzeugbar sind, in einer gemeinsamen optischen Einheit verwendbar. Damit werden die Selektivität erhöht und Störungen reduziert. Es ist aber auch möglich, in der optischen Einheit nur chemilumineszierende Indikatorsubstanzen zu verwenden. Strahlungsquellen für Anregungsstrahlung müssen dann nicht angeordnet oder genutzt werden.

In jedem der beispielhaft dargestellten Fälle wird stets die Bedingung eingehalten, dass die Beaufschlagung der Eintrittsfläche mit mehreren Emissionen (bei gleichen oder unterschiedlichen Emissionswellenlängen) nicht zum selben Zeitpunkt am gleichen Ort erfolgt. Für die Beaufschlagung der Empfangsebene und/oder einer Eintrittsfläche einer wellenlängenselektiven Einrichtung mit Emissionen an einem Ort oder verschiedenen Orten und/oder zu verschiedenen Zeiten werden unter anderem Abbildungssysteme, Strahlformer und/oder Taktgeber geeignet angeordnet und betrieben.

Die Offenbarung dieser Anmeldung beschreibt somit außerhalb des Schutzumfangs der Ansprüche eine Vorrichtung zur Erzeugung und Messung der Emission eines Indikators, der einen Träger und mindestens eine mit Analyten (590) beaufschlagbare Indikatorsubstanz aufweist, mit mindestens einer Strahlungsquelle für Anregungsstrahlung und mindestens einem Empfänger für die Messung der Emission, wobei die Vorrichtung dadurch gekennzeichnet ist, dass eine oder mehrere Strahlungsquellen mit quasi-monochromatischer Anregungsstrahlung an einem oder verschiedenen Orten in einer Strahlungsfläche angeordnet sind, der Indikator mit mehreren Indikatorsubstanzen in einer Indikatorfläche an verschiedenen Orten und/oder zu verschiedenen Zeiten zur Erzeugung der Emission mit Anregungsstrahlung beaufschlagbar ist, für einen oder verschiedene Orte einer Empfangsfläche die Emission empfangbar ist, die Eintrittsfläche einer wellenlängenselektiven Einrichtung mit der in der Empfangsfläche empfangenen Emission beaufschlagbar ist und in einer Austrittsfläche der wellenlängenselektiven Einrichtung ein oder mehrere Empfänger für den Empfang von Emissionen angeordnet sind, wobei die Strahlungsfläche, die Indikatorfläche und die Empfangsfläche definiert beabstandet sind und die Vorrichtung so konfiguriert ist, dass die Emissionen in der Eintrittsfläche der wellenlängenselektiven Einrichtung örtlich und/oder zeitlich derart entkoppelt sind, dass die Emissionen durch die wellenlängenselektive Einrichtung ohne gegenseitige Überlagerung von Emissionen auflösbar sind.

Die optische Vorrichtung zur Erzeugung und Messung von Emissionen befindet sich vorzugsweise bei betriebsfertig zusammengebauten Geräten innerhalb eines nicht näher dargestellten robusten Gehäuses mit Display. Ein angekoppelter Indikator kann sich im gleichen Gehäuse oder in einem separaten, vom Gehäuse abnehmbaren eigenen Gehäuse befinden. In dem Gehäuse können auch eine Adsorptions- und Desorptionsvorrichtung zur Führung von Analyten, Elektronik sowie eine autonome, nicht leitungsgebundene Energieversorgung (Batterie oder Akku) aufgenommen sein. Bei bevorzugten Ausführungsformen ist das komplette System als leicht transportierbares, kompaktes handgehaltenes (handheld) Gerät ausgebildet, das weitestgehend unanfällig gegenüber Störungen durch Stoß und Vibrationen ist. Es ist staub- und wasserdicht und über einen großen Temperaturbereich betriebssicher zu betreiben.

## Patentansprüche

1. Portables Gerät (100) zur Detektion von Explosivstoffen mit einer Vorrichtung (200) zur Erzeugung und Messung von Emission eines Indikators (120),
wobei der Indikator einen Träger (122) und mehrere mit Analyten (190) beaufschlagbare Indikatorsubstanzen (125-1, 125-2) aufweist, die als dünne Schicht auf dem Träger (122) aufgebracht sind und mittels einer Haltevorrichtung (180) in einer Indikatorfläche (130) positionierbar sind, wobei die Indikatorsubstanzen (125-1, 125-2) in unterschiedlichen Bereichen des Trägers (122) derart angeordnet sind, dass die Indikatorsubstanzen in der Indikatorfläche (130) ein Muster von Indikatorsubstanzen in Abhängigkeit vom Ort darstellen, wobei die Vorrichtung (200) umfasst:
mehrere Strahlungsquellen (210-1, 210-2) zur Abgabe quasi-monochromatischer Anregungsstrahlung, wobei die Strahlungsquellen an verschiedenen Orten in einer Strahlungsfläche (240) derart angeordnet sind, dass die Strahlungsfläche ein Muster von Strahlungsquellen (210-1, 210-2) in Abhängigkeit vom Ort darstellt;
einem Anregungsstrahlengang mit mindestens einem ersten Abbildungssystem (220-1, 220-2) zur Abbildung von Anregungsstrahlung in die Indikatorfläche (130) derart, dass Emission der Indikatorsubstanzen an den Orten erzeugbar ist, an denen die Anregungsstrahlung auf der Indikatorfläche abgebildet wird;
einem Emissionsstrahlengang mit mindestens einem zweiten Abbildungssystem (250) zur Abbildung der Indikatorfläche in eine Empfangsfläche (245) derart, dass in der Empfangsfläche ein Muster von Emissionen in Abhängigkeit vom Ort erzeugbar ist;
mehrere Empfänger (270-1, 270-2) für den Empfang von Emissionen aus der Empfangsfläche und zur Wandlung der empfangenen Emissionen in elektrische Signale,
wobei der Anregungsstrahlengang und der Emissionsstrahlengang in Rückwärtsgeometrie angeordnet sind, so dass der Anregungsstrahlengang und der Emissionsstrahlengang auf ein und derselben Seite der Indikatorfläche (130) angeordnet sind, und
wobei der Anregungsstrahlengang mehrere Anregungskanäle aufweist, wobei jeder Anregungskanal ein erstes Abbildungssystem (220-1, 220-2) aufweist, das eine optische Achse (222-1, 222-2) des Anregungskanals definiert,
**dadurch gekennzeichnet, dass**
die optischen Achsen (222-1, 222-2, 252) der ersten Abbildungssysteme (220-1, 220-2) des Anregungsstrahlengangs und von einem oder mehreren zweiten Abbildungssystemen (250) des Emissionsstrahlengangs sich mit Abstand vor der Indikatorfläche (130) kreuzen, so dass Durchstoßpunkte der optischen Achsen (222-1, 222-2, 252) in der Indikatorfläche (130) lateral auseinanderliegen.

2. Gerät nach Anspruch 1, **dadurch gekennzeichnet, dass** der Anregungsstrahlengang zwischen zwei und zehn, insbesondere drei bis sechs separate Anregungskanäle aufweist und/oder dass für jeden Indikatorbereich genau ein Anregungskanal vorgesehen ist, so dass die Anzahl der Anregungskanäle der Anzahl der Indikatorbereiche entspricht.

3. Gerät nach einem der vorhergehenden Ansprüche, **dadurch gekennzeichnet, dass** der Emissionsstrahlengang für alle Emissionen ein gemeinsames zweites Abbildungssystem (250) aufweist, das eine optische Achse (252) des Emissionsstrahlengangs definiert.

4. Gerät nach einem der vorhergehenden Ansprüche, **dadurch gekennzeichnet, dass** die Anregungskanäle in Bezug auf den Emissionskanal eine symmetrische Anordnung bilden, wobei die Anordnung eine Spiegelsymmetrie zu mindestens einer die optische Achse (252) des Emissionsstrahlengangs enthaltenden Symmetrieebene aufweist, wobei vorzugsweise die Anregungskanäle und der Emissionskanal eine kegelförmige Anordnung bilden, wobei optische Achsen (222-1, 222-2) der Anregungskanäle auf einer Kegelmantelfläche angeordnet sind, die den Emissionskanal umgibt, und die optische Achse (252) des Emissionskanals in der Achse des Kegels angeordnet ist.

5. Gerät nach einem der vorhergehenden Ansprüche, **dadurch gekennzeichnet, dass** eine Messgeometrie so eingestellt ist, dass vom Indikator (120) spekular reflektierte Anregungsstrahlung nicht auf die Empfangsfläche (245) trifft, wobei unterschiedliche Winkel von Anregungsstrahlung und Emissionsstrahlung zur Oberflächennormalen des Indikators im Bereich des Auftreffens der Anregungsstrahlung vorgesehen sind.

6. Gerät nach einem der vorhergehenden Ansprüche, **dadurch gekennzeichnet, dass** optische Achsen (222-1, 222-2, 252) der ersten Abbildungssysteme (220-1, 220-2) des Anregungsstrahlengangs und des mindestens einen zweiten Abbildungssystem (250) des Emissionsstrahlengangs einen Winkel einschließen, wobei der Winkel vorzugsweise im Bereich von 20° bis 60°, insbesondere im Bereich von 30° bis 50° liegt.

7. Gerät nach einem der vorhergehenden Ansprüche, **dadurch gekennzeichnet, dass** jeder Indikatorsubstanz (125-1, 125-2) ein separater Empfänger (270-1, 270-2) zugeordnet ist und/oder dass zwischen den Empfängern (270-1, 270-2) Zwischenräume ohne lichtempfindliche Flächen liegen
und/oder dass die Empfänger (270-1, 270-2) individuell justierbar sind und/oder dass jeder Indikatorsubstanz (125-1, 125-2) eine oder mehrere Strahlungsquellen (210-1, 210-2) zugeordnet sind, wobei eine Strahlungsquelle höchstens einer Indikatorsubstanz zugeordnet ist, so dass es keine zwei oder mehr Indikatorsubstanzen gibt, denen dieselbe Strahlungsquelle zugeordnet ist.

8. Gerät nach einem der vorhergehenden Ansprüche, **dadurch gekennzeichnet, dass** in einem Anregungskanal zwischen einer Strahlungsquelle und der Indikatorebene ein Strahlformer (326) angeordnet ist, wobei vorzugsweise der Strahlformer dafür ausgelegt ist, ein ungleichmäßiges Intensitätsprofil in ein gleichmäßiges und steiles Intensitätsprofil umzuwandeln.

9. Gerät nach einem der vorhergehenden Ansprüche, **gekennzeichnet durch** einen Taktgeber (320) zum Ansteuern von Strahlungsquellen (210-1, 210-2), wobei der Taktgeber derart konfiguriert ist, dass alle Strahlungsquellen seriell nacheinander oder dass ein Teil der Strahlungsquellen gleichzeitig getaktet wird oder dass alle Strahlungsquellen gleichzeitig getaktet werden.

10. Gerät nach einem der vorhergehenden Ansprüche, **gekennzeichnet durch** mindestens einem Anregungskanal, dem zwei oder mehr Strahlungsquellen (310-1, 310-2) zur Abgabe unterschiedlicher Anregungswellenlängen zugeordnet sind, wobei die Strahlungsquellen mittels eines Taktgebers (320) wechselweise taktbar sind und eine Strahlungsvereinigungseinrichtung (328) zur wahlweisen Einkopplung von Anregungsstrahlung der Strahlungsquellen in den Anregungskanal vorgesehen ist, wobei die Strahlungsvereinigungseinrichtung vorzugsweise einen als Diffusor wirkenden Strahlformer (326) und eine Lochblende (327) aufweist.

11. Gerät nach einem der vorhergehenden Ansprüche, **dadurch gekennzeichnet, dass** alle optischen Elemente des Abbildungsstrahlengangs und des Emissionsstrahlengangs in einem einzigen Bauteil (290) integriert sind, das als Optikhalter für die optischen Elemente fungiert.

12. Gerät nach einem der vorhergehenden Ansprüche, **gekennzeichnet durch** eine an die Empfänger angeschlossene Auswerteeinrichtung zum Empfang von elektrischen Signalen der Empfänger und zur Auswertung der in den Signalen enthaltenen spektralen Information über die Emissionen, wobei die Auswerteeinrichtung so konfiguriert ist, dass mindestens eine der folgenden Informationen ermittelbar und an einen Bediener ausgebbar ist:
Informationen über die Menge einer oder mehrerer gesuchter Analyten,
Information über die Art einer oder mehrerer Analyten,
wobei die Analyten einen oder mehrere Explosivstoffe umfassen.

13. Gerät nach einem der vorhergehenden Ansprüche, **gekennzeichnet durch** mindestens eines der folgenden Merkmale:
das Gerät ist als Handheld-Gerät ausgelegt, so dass das Gerät bei der Benutzung mit nur einer Hand gehalten werden kann;
das Gerät hat ein Gewicht von weniger als 2 Kilogramm, insbesondere weniger als 1.5 Kilogramm;
das Gerät hat inklusive der Halteeinrichtung für den Indikator eine Baugröße von weniger als 50 cm, insbesondere weniger als 40 cm, jeweils gemessen in der Richtung größter Ausdehnung;
die Haltevorrichtung ist in Bezug auf die Vorrichtung (200) derart angebracht, dass die auf dem Träger des Indikators angebrachte dünne Schicht mit Indikatorsubstanzen (125-1, 125-2) in der Indikatorfläche (130) positioniert ist,
wobei vorzugsweise die Haltevorrichtung (180) einen Zuluftanschluss (182) mit einer Einlassöffnung zum Ansaugen eines gasförmigen Mediums aufweist, das den oder die gesuchten Analyten trägt, wobei das Medium mit den Analyten mithilfe eines Fluidkanalsystems über die Indikatorsubstanzen (125-1, 125-2) führbar und nach Kontakt mit diesen über einen Abluftanschluss (184) aus der Haltevorrichtung (180) ausgebbar ist, wobei die Haltevorrichtung vorzugsweise eine Heizeinrichtung (185) zum Aufheizen des angesaugten Mediums vor Kontakt mit den Indikatorsubstanzen aufweist.

14. Verwendung eines Geräts nach einem der vorhergehenden Ansprüche zur Detektion von Explosivstoffen, wobei ein Indikator verwendet wird, der mindestens eine Indikatorsubstanz aufweist, die bei Bestrahlung mit Anregungsstrahlung und Kontakt mit einem mindestens einen Explosivstoff enthaltenden Analyten eine Verminderung oder Verstärkung der von der Indikatorsubstanz abgegebenen Fluoreszenzintensität zeigt.

## Claims

1. Portable appliance (100) for detecting explosive materials, having an apparatus (200) for generating and measuring emission of an indicator (120),
wherein the indicator has a carrier (122) and a plurality of indicator substances (125-1, 125-2) that can be impinged upon by analytes (190), said indicator substances being applied onto the carrier (122) as a thin layer and being positionable in an indicator area (130) by means of a holding apparatus (180), wherein the indicator substances (125-1, 125-2) are arranged in different regions of the carrier (122) in such a way that the indicator substances in the indicator area (130) represent a pattern of indicator substances depending on the location, wherein the apparatus (200) comprises:
a plurality of radiation sources (210-1, 210-2) for emitting quasi-monochromatic excitation radiation, wherein the radiation sources are arranged at various locations in a radiation area (240) in such a way that the radiation area represents a pattern of radiation sources (210-1, 210-2) depending on the location;
an excitation beam path with at least one first imaging system (220-1, 220-2) for imaging excitation radiation into the indicator area (130) in such a way that emission of the indicator substances is producible at the locations at which the excitation radiation is imaged on the indicator area;
an emission beam path having at least one second imaging system (250) for imaging the indicator area into a reception area (245) in such a way that a pattern of emissions is producible in the reception area depending on the location;
a plurality of receivers (270-1, 270-2) for receiving emissions from the reception area and for converting the received emissions into electrical signals
wherein the excitation beam path and the emission beam path are arranged in a backward geometry such that the excitation beam path and the emission beam path are arranged on one and the same side of the indicator area (130), and
wherein the excitation beam path has a plurality of excitation channels, wherein each excitation channel has a first imaging system (220-1, 220-2) that defines an optical axis (222-1, 222-2) of the excitation channel,
**characterized in that**
the optical axes (222-1, 222-2, 252) of the first imaging systems (220-1, 220-2) of the excitation beam path and of one or more second imaging systems (250) of the emission beam path cross at a distance in front of the indicator area (130), and so points of intersection of the optical axes (222-1, 222-2, 252) in the indicator area (130) are laterally spaced apart.

2. Appliance according to Claim 1, **characterized in that** the excitation beam path comprises between two and ten, in particular three to six, separate excitation channels and/or **in that** exactly one excitation channel is provided for each indicator region such that the number of excitation channels corresponds to the number of indicator regions.

3. Appliance according to either of the preceding claims, **characterized in that** the emission beam path has a common second imaging system (250) for all emissions, said second imaging system defining an optical axis (252) of the emission beam path.

4. Appliance according to any of the preceding claims, **characterized in that** the excitation channels form a symmetric arrangement in relation to the emission channel, wherein the arrangement is mirror symmetric in relation to at least one symmetry plane that contains the optical axis (252) of the emission beam path,
wherein preferably the excitation channels and the emission channel form a conical arrangement, wherein optical axes (222-1, 222-2) of the excitation channels are arranged on a lateral conical face that surrounds the emission channel and the optical axis (252) of the emission channel is arranged along the axis of the cone

5. Appliance according to any of the preceding claims, **characterized in that** a measurement geometry is set in such a way that excitation radiation that has undergone specular reflection at the indicator (120) does not strike the reception area (245), wherein different angles of excitation radiation and emission radiation in relation to the surface normal of the indicator are provided in the region where the excitation radiation strikes.

6. Appliance according to any of the preceding claims, **characterized in that** optical axes (222-1, 222-2, 252) of the first imaging systems (220-1, 220-2) of the excitation beam path and of the at least one second imaging system (250) of the emission beam path include an angle, wherein the angle preferably lies in the range from 20° to 60°, in particular in the range from 30° to 50°.

7. Appliance according to any of the preceding claims, **characterized in that** a separate receiver (270-1, 270-2) is assigned to each indicator substance (125-1, 125-2)
and/or **in that** interstices without light-sensitive areas lie between the receivers (270-1, 270-2)
and/or **in that** the receivers (270-1, 270-2) are individually adjustable
and/or **in that** one or more radiation sources (210-1, 210-2) are assigned to each indicator substance (125-1, 125-2), wherein a radiation source is assigned to at most one indicator substance, and so there are not two or more indicator substances that are assigned the same radiation source.

8. Appliance according to any of the preceding claims, **characterized in that** a beam shaper (326) is arranged in an excitation channel between a radiation source and the indicator plane, wherein preferably the beam shaper is designed to convert a non-uniform intensity profile into a uniform and steep intensity profile.

9. Appliance according to any of the preceding claims, **characterized by** a clock (320) for actuating radiation sources (210-1, 210-2), wherein the clock is configured in such a way that all radiation sources are clocked sequentially in series or that some of the radiation sources are clocked simultaneously or that all radiation sources are clocked simultaneously.

10. Appliance according to any of the preceding claims, **characterized by** at least one excitation channel that is assigned two or more radiation sources (310-1, 310-2) for emitting different excitation wavelengths, wherein the radiation sources are alternately clockable by means of a clock (320) and a radiation merging device (328) is provided for selective input coupling of excitation radiation from the radiation sources into the excitation channel, wherein the radiation merging device preferably has a beam shaper (326) that acts as a diffuser and a pinhole diaphragm (327).

11. Appliance according to any of the preceding claims, **characterized in that** all optical elements of the imaging beam path and of the emission beam path are integrated in a single component (290) that acts as an optics holder for the optical elements.

12. Appliance according to any of the preceding claims, **characterized by** an evaluation device, connected to the receivers, for receiving electrical signals of the receivers and for evaluating the spectral information about the emissions contained in the signals, wherein the evaluation device is configured in such a way that at least one of the following information items is establishable and can be output to an operator:
information items about the amount of one or more searched-for analytes
information about the type of one or more analytes, wherein the analytes comprise one or more explosive materials.

13. Appliance according to any of the preceding claims, **characterized by** at least one of the following features:
the appliance is designed as a hand-held appliance such that the appliance can be held by only one hand during use;
the appliance has a mass of less than 2 kilograms, in particular less than 1.5 kilograms;
the appliance has an installation size, including the holding device for the indicator, of less than 50 cm, in particular of less than 40 cm, respectively measured in the direction of greatest extent;
the holding apparatus is attached in relation to the apparatus (200) in such a way that the thin layer with indicator substances (125-1, 125-2) that is applied to the carrier of the indicator is positioned in the indicator area (130),
wherein preferably the holding apparatus (180) has a delivery air connector (182) with an inlet opening for suctioning a gaseous medium that carries the searched-for analyte or analytes, wherein the medium with the analytes can be guided over the indicator substances (125-1, 125-2) with the aid of a fluid channel system and can be output from the holding apparatus (180) after contact with said indicator substances by way of an exhaust air connector (184), wherein the holding apparatus preferably has a heating device (185) for heating the suctioned medium prior to contact with the indicator substances.

14. Use of an appliance according to any of the preceding claims for detecting explosive materials, wherein use is made of an indicator that has at least one indicator substance which, in the case of irradiation by excitation radiation and contact with an analyte containing at least one explosive material, exhibits a reduction or increase in the fluorescence intensity emitted by the indicator substance.

## Revendications

1. Appareil portatif (100) permettant de détecter des explosifs à l'aide d'un dispositif (200) pour générer et mesurer l'émission d'un indicateur (120),
dans lequel l'indicateur présente un support (122) et plusieurs substances indicatrices (125-1, 125-2) pouvant être soumises à des analytes (190) et qui sont appliquées en couche mince sur le support (122) et peuvent être positionnées dans une surface indicatrice (130) au moyen d'un dispositif de retenue (180), dans lequel les substances indicatrices (125-1, 125-2) sont disposées dans différentes zones du support (122) de telle sorte que les substances indicatrices représentent dans la surface indicatrice (130) un motif de substances indicatrices en fonction de l'emplacement, le dispositif (200) comprenant:
plusieurs sources de rayonnement (210-1, 210-2) pour émettre un rayonnement d'excitation quasi-monochromatique, dans lequel les sources de rayonnement sont disposées à différents emplacements dans une surface de rayonnement (240) de telle sorte que la surface de rayonnement représente un motif de sources de rayonnement (210-1, 210-2) en fonction de l'emplacement;
une trajectoire de faisceau d'excitation pourvue d'au moins un premier système de formation d'image (220-1, 220-2) pour former une image d'un rayonnement d'excitation dans la surface indicatrice (130) de telle sorte que l'émission des substances indicatrices peut être générée aux emplacements où une image du rayonnement d'excitation est formée sur la surface indicatrice;
une trajectoire de faisceau d'émission pourvue d'au moins un deuxième système de formation d'image (250) pour former une image de la surface indicatrice dans une surface de réception (245) de telle sorte qu'un motif d'émissions peut être généré dans la surface de réceptionen fonction de l'emplacement;
plusieurs récepteurs (270-1, 270-2) pour recevoir des émissions à partir de la surface de réception et pour convertir les émissions reçues en signaux électriques,
dans lequel la trajectoire de faisceau d'excitation et la trajectoire d'émission sont disposées dans une géométrie inverse de sorte que la trajectoire de faisceau d'excitation et la trajectoire de faisceau d'émission sont disposées sur un seul et même côté de la surface indicatrice (13(, et
dans lequel la trajectoire de faisceau d'excitation présente plusieurs canaux d'excitation chaque canal d'excitation présentant un premier système de formation d'image (220-1, 220-2) qui définit un axe optique (222-1, 222-2) du canal d'excitation
**caractérisé en ce que** les axes optiques (222-1, 222-2, 252) des premiers systèmes de formation d'image (220-1, 220-2) de la trajectoire de faisceau d'excitation et d'un ou de plusieurs deuxièmes systèmes de formation d'image (250) de la trajectoire de faisceau d'émission se croisent à distance devant la surface indicatrice (130) de sorte que des points de percée des axes optiques (222-1, 222-2, 252) sont écartés latéralement dans la surface indicatrice(130).

2. Appareil selon la revendication 1, **caractérisé en ce que** la trajectoire de faisceau d'excitation présente entre deux et dix, en particulier de trois à six canaux d'excitation séparés, et/ou **en ce qu'**un canal d'excitation exactement est prévu pour chaque zone indicatrice de sorte que le nombre des canaux d'excitation correspond au nombre des zones indicatrices

3. Appareil selon l'une quelconque des revendications précédentes, **caractérisé en ce que** la trajectoire de faisceau d'émission présente pour toutes les émissions un deuxième système de formation d'image (250) commun qui définit un axe optique (252) de la trajectoire de faisceau d'émission

4. Appareil selon l'une quelconque des revendications précédentes, **caractérisé en ce que** les canaux d'excitation constituent un agencement symétrique par rapport au canal d'émission, l'agencement présentant une symétrie miroir par rapport à au moins un plan de symétrie contenant l'axe optique (252) de la trajectoire de faisceau d'émission
dans lequel de préférence les canaux d'excitation et le canal d'émission constituent un agencement conique, les axes optiques (222-1, 222-2) des canaux d'excitation étant disposés sur une surface latérale de cône qui entoure le canal d'émission, et l'axe optique (252) du canal d'émission est disposé dans l'axe du cône.

5. Appareil selon l'une quelconque des revendications précédentes, **caractérisé en ce qu'**une géométrie de mesure est réglée de telle sorte qu'un rayonnement d'excitation réfléchi de manière spéculaire par l'indicateur (120) n'est pas incident sur la surface de réception (245), dans lequel différents angles du rayonnement d'excitation et du rayonnement d'émission par rapport à la normale à la surface de l'indicateur sont prévus dans la zone d'incidence du rayonnement d'excitation

6. Appareil selon l'une quelconque des revendications précédentes, **caractérisé en ce que** les axes optiques (222-1, 222-2, 252) des premiers systèmes de formation d'image (220-1, 220-2) de la trajectoire de rayonnement d'excitation et dudit au moins un deuxième système de formation d'image (250) de la trajectoire de faisceau d'émission définissent un angle, l'angle étant de préférence compris dans la plage de 20° à 60°, en particulier dans la plage de30° à 50°.

7. Appareil selon l'une quelconque des revendications précédentes, **caractérisé en ce qu'**un récepteur séparé (270-1, 270-2) est associé à chaque substance indicatrice (125-1, 125-2), et/ou **en ce que** des espaces sans surfaces photosensibles se trouvent entre les récepteurs (270-1, 270-2),
et/ou **en ce que** les récepteurs (270-1, 270-2) sont ajustables individuellement
et/ou **en ce qu'**une ou plusieurs sources de rayonnement (210-1, 210-2) sont associées à chaque substance indicatrice (125-1, 125-2), dans lequel une source de rayonnement est associée au maximum à une substance indicatrice de sorte qu'il n'existe jamais deux ou plusieurs substances indicatrices auxquelles est associée la même source de rayonnement

8. Appareil selon l'une quelconque des revendications précédentes, **caractérisé en ce qu'**un formateur de faisceau (326) est disposé dans un canal d'excitation entre une source de rayonnement et le plan d'indicateur, dans lequel de préférence le formateur de faisceau est conçu pour convertir un profil d'intensité irrégulier en un profil d'intensité régulier et à forte pente.

9. Appareil selon l'une quelconque des revendications précédentes, **caractérisé par** une horloge (320) pour piloter des sources de rayonnement (210-1, 210-2), l'horloge étant configurée de telle sorte que toutes les sources de rayonnement sont cadencées en série les unes après les autres, ou en ce qu'une partie des sources de rayonnement est cadencée en même temps, ou en ce que toutes les sources de rayonnement sont cadencées en même temps

10. Appareil selon l'une quelconque des revendications précédentes, **caractérisé par** au moins un canal d'excitation auquel sont associées deux ou plusieurs sources de rayonnement (310-1, 310-2) pour émettre différentes longueurs d'onde d'excitation, dans lequel les sources de rayonnement peuvent être cadencées en alternance au moyen d'une horloge (320), et un dispositif de concentration de rayonnement (328) pour une injection sélective d'un rayonnement d'excitation des sources de rayonnement dans le canal d'excitation est prévu, le dispositif de concentration de rayonnement présentant de préférence un formateur de faisceau (326) agissant comme un diffuseur et un diaphragme à trou (327)

11. Appareil selon l'une quelconque des revendications précédentes, **caractérisé en ce que** tous les éléments optiques de la trajectoire de formation d'image et de la trajectoire d'émission sont intégrés dans un seul composant (290) qui sert de support d'optique pour les éléments optiques

12. Appareil selon l'une quelconque des revendications précédentes, **caractérisé par** un dispositif d'évaluation connecté aux récepteurs pour recevoir des signaux électriques des récepteurs et pour évaluer l'information spectrale contenue dans les signaux concernant les émissions, dans lequel le dispositif d'évaluation est configuré de telle sorte qu'au moins l'une des informations suivantes peut être établie et sortie pour un utilisateur:
des informations concernant la quantité d'un ou de plusieurs analytes recherchés,
des informations concernant le type d'un ou de plusieurs analytes,
les analytes comprenant un ou plusieurs explosifs

13. Appareil selon l'une quelconque des revendications précédentes, **caractérisé par** au moins l'une des particularités suivantes:
l'appareil est conçu comme un appareil portable de sorte que l'appareil peut être tenu d'une seule main par l'utilisateur pendant l'utilisation;
l'appareil présente un poids inférieur à 2 kilogrammes, en particulier inférieur à 1,5 kilogrammes ;
l'appareil avec son dispositif de retenue pour l'indicateur présente un format inférieur à 50 cm, en particulier inférieur à 40 cm, respectivement mesuré dans la direction de sa plus grande dimension ;
le dispositif de retenue est fixé par rapport au dispositif (200) de telle sorte que la couche mince appliquée sur le support de l'indicateur avec des substances indicatrices (125-1, 125-2) est positionnée dans la surface indicatrice(130),
dans lequel de préférence le dispositif de retenue (180) présente une prise d'air frais (182) pourvue d'une ouverture d'entrée pour aspirer un milieu gazeux qui porte le ou les analytes recherchés, dans lequel le milieu avec les analytes peut être amené à l'aide d'un système de canaux de fluide sur les substances indicatrices (125-1, 125-2), et après un contact avec celles-ci, peut être sorti du dispositif de retenue (180) par l'intermédiaire d'une prise d'air vicié (184), dans lequel le dispositif de retenue présente de préférence un dispositif de chauffage (185) pour chauffer le milieu aspiré avant le contact avec les substances indicatrices

14. Utilisation d'un appareil selon l'une quelconque des revendications précédentes pour détecter des explosifs, dans laquelle un indicateur est utilisé qui présente au moins une substance indicatrice qui indique sous rayonnement avec un rayonnement d'excitation et en cas de contact avec un analyte contenant au moins un explosif une diminution ou une augmentation de l'intensité de fluorescence émise par la substance indicatrice.
